(19) Europäisches Patentamt
European Patent Office
Office européen des brevets

(11) Publication number: **0 450 585 A2**

(12) **EUROPEAN PATENT APPLICATION**

(21) Application number: **91105231.4**

(22) Date of filing: **03.04.91**

(51) Int. Cl.5: **C07H 3/08**, C07H 5/00, C07H 11/00, C07H 13/00, C07H 13/02, C07H 13/12, C07H 15/04, C07H 15/207, C07H 17/04, C07H 19/06, C07H 19/16

(30) Priority: **04.04.90 AT 791/90**
**03.07.90 AT 1410/90**

(43) Date of publication of application:
**09.10.91 Bulletin 91/41**

(84) Designated Contracting States:
**AT BE CH DE DK ES FR GB GR IT LI LU NL SE**

(71) Applicant: **CHEMPROSA, CHEMISCHE PRODUKTE SAISCHEK GmbH**
**Dr. Robert-Graf-Strasse 15**
**A-8010 Graz(AT)**

(72) Inventor: **Saischek, Gerald, Dipl.-Ing.**
**Moserhofgasse 50**
**A-8010 Graz(AT)**
Inventor: **Fuchs, Franz**
**Erlenweg 4**
**A-8141 Unterpremstätten(AT)**
Inventor: **Dax, Karl, Dr.**
**Ludwig-Werba-Gasse 16**
**A-8010 Graz(AT)**
Inventor: **Billiani, Gertrude, Dr.**
**Kasernstrasse 76/1/4**
**A-8041 Graz(AT)**

(74) Representative: **TER MEER - MÜLLER - STEINMEISTER & PARTNER**
**Mauerkircherstrasse 45**
**W-8000 München 80(DE)**

(54) **Process for the manufacture of 2-deoxy-D-threo-pentofuranosides, intermediates for their manufacture and their use.**

(57) Disclosed is a process for the manufacture of 2-Deoxy-D-threo-pentofuranosides of formula 1

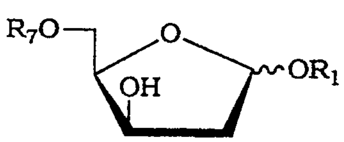
(1)

wherein $R_1$ is an alkyl group having 1 to 4 carbon atoms and $R_7$ is a hydroxy protection group by reacting a 2-deoxy-D-erythro-pentofuranoside of formula 2

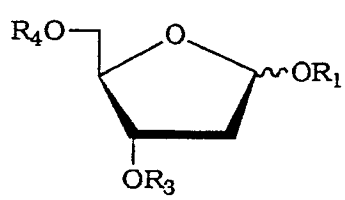
(2)

wherein $R_1$ is an alkyl group having 1 to 4 carbon atoms, $R_3$ is an alkyl-, aryl-, alkylaryl- or aralkyl-sulfonyl group, either unsubstituted or substituted one or more times by halogen atoms, nitro or alkoxy groups, an imidazolesulfonyl or halosulfonyl group, and $R_4$ has the same meaning as $R_3$ or signifies a hydroxy protection group.

Furtheron disclosed are a process for the manufacture of 3-substituted 2-deoxy-D-erythro-pentofuranosides using these compounds and novel and substituted and unsubstituted 2-deoxy-D-pen-

EP 0 450 585 A2

tofuranosides and their use for the manufacture of 3'-substituted 2'-deoxynucleosides.

The subject matter of the present invention is a process for the manufacture of 2-deoxy-D-threo-pentofuranose derivatives, a process for the manufacture of 3-substituted 2-deoxy-D-erythro-pentofuranose derivatives using these compounds, novel substituted and unsubstituted 2-deoxy-pentofuranosesand their use for the manufacture of 3'-substituted 2-deoxynucleosides.

3'-Azido-2',3'-dideoxynucleosides which are pharmaceutically interesting compounds can be manufactured

a) by introducing an azide residue in the 3'-position and, if necessary, deoxygenating in the 2'-position of a nucleoside, or

b) by manufacturing 3-azido-2,3-dideoxy-D-erythro-pentofuranoses and their condensation with a N-heterocyclic base to give the nucleosides.

Processes according to method (a) are given for example by Tai-Shun et al., (J. Med. Chem., 1983, 26, 544-548); J.P. Horwitz et al., (J. Org. Chem., 1964, 29, 2076-2078); T.A. Krenitsky et al., (J. Med. Chem., 1983, 26, 891-895); N.C. Miller et al., (J. Org. Chem., 1964, 29, 1772-1776); G. Etzold, (Tetrahedron, 1971, 27, 2463-2472) and J.J. Fox et al., (J. Org. Chem., 1963, 28, 936-942). For this method the very expensive thymidine or 2'-deoxyuridine have to be used as the starting material. These processes proceed with a double inversion of configuration on the 3'-C atom. Since this double configuration change is carried out in the finished nucleoside and due to the use of strong bases, secondary products arise and the results are unsatisfactory with regard to the yield for a technical process, so that there is a pressing need for the development of an efficient manufacturing process for 3'-azidonucleosides.

The manufacture of 3-azido-2,3-dideoxy-D-erythropentofuranoses for condensation with bases according to method (b) was previously possible only by tedious ways. These processes are described in G.W.J. Fleet et al., (Tetrahedron, 1988, 44, 625-636); M.K. Gurjar et al., (Indian Journal of Chemistry, 1987, 26B, 905); N.B. Dyatkina et al., (Synthesis, 1984, 961-963), and start from D-xylose. For this, all processes start with deoxygenation in position 2, using tributyl tin hydride, and then the introduction of azide into position 3. However, the deoxygenation and processing by the tributyl tin hydride reaction according to Fleet et al., (Tetrahedron, 1988, 44, page 626, line 25 et seq.) is very difficult and the yields are unsatisfactory.

Chung K. Chu et al. have described the total synthesis of 3'-azido-3'-deoxythymidine and of 3'-azido-2',3'-dideoxyuridine (Tetrahedron Letters, 1988, 42, 5349-5352). Due to the very expensive reagents used and the 10 steps involved, the synthesis is also unsuitable for the production on a technical scale.

2',3'-Dideoxy-3'-fluoro-nucleosides can be obtained

c) by fluorination at the 3' position and, if required, deoxygenation at at the 2' position of a nucleoside, or

d) by preparation of 2,3-dideoxy-3-fluoro-D-erythro-pentofuranoses and their condensation with a N-heterocyclic base to give the nucleosides.

The process according to method c) can be carried out for example according to P. Herdewijn et al., (J. Med. Chem., 1987, 30, 1270-1278). Here, however, the fluorination at position 3' is only achieved with the aid of diethylaminosulphur trifluoride (DAST).

The preparation of 2,3-dideoxy-3-fluoro-D-erythro-pentofuranoses for condensation with a purine or pyrimidine base according to method d) has so far been either laborious or only possible using starting materials that were difficult to obtain.

Thus P. Bravo et al., (J. Org. Chem., 1989, 54, 5171), describe the use of (R)-1-fluoro-3((4-methylphenyl)sulfinyl)acetone for the preparation of fluorodideoxyfuranoses in a multistage total synthesis.

According to Chemical Abstracts 1989, 111, 233479p the preparation of 2,3-dideoxy-3-fluoro-D-erythro-pentofuranose derivatives involves the use of 2-deoxy-D-threopentofuranose derivatives, but details of the preparation of the latter derivatives are not disclosed.

Fleet et al., (Tetrahedron, 1988, 44, 625-636), describe the preparation of 2,3-dideoxy-3-fluoro-D-erythro-pentofuranose derivatives from D-xylose by deoxygenation at position 2 with use of tributyltin hydride and subsequent fluorination at position 3. According to Fleet et al. (p. 626, line 25 et seq.), however, the deoxygenation and working up after the tributyltin hydride reaction are difficult and the yields unsatisfactory.

The object of the present invention therefore is the provision of a process with which substituted and unsubstituted 3-substituted 2-deoxy-D-erythro-pentofuranose derivatives can be obtained easily and with a high yield starting from the readily accessible 2-deoxy-D-erythro-pentose (2-deoxy-D-ribose), whereby the number of required synthetic steps is reduced as well as the deoxygenation reaction is avoided, leading to high yields.

Subject matter of the present invention therefore is a process for the manufacture of 2-deoxy-D-threo-pentofuranosides of formula 1

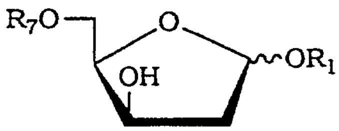

(1)

wherein $R_1$ is an alkyl group having 1-4 carbon atoms and $R_7$ is a hydroxy protection group, which is characterized in that a 2-deoxy-D-erythro-pentofuranoside of formula 2

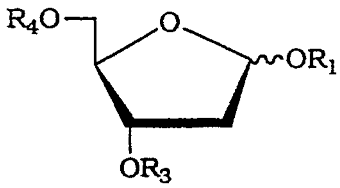

(2)

wherein $R_1$ is an alkyl group having 1 to 4 carbon atoms, $R_3$ is an alkyl-, aryl-, alkylaryl- or aralkylsulfonyl group, either unsubstituted or substituted one or more times by halogen atoms, nitro or alkoxy groups, an imidazolesulfonyl or halosulfonyl group, and $R_4$ has the same meaning as $R_3$ or signifies a hydroxy protection group,
is reacted either:

a) if $R_4$ in formula 2 has the same meaning as $R_3$, with at least two equivalents of an ionogenic nitrite in a dilution solvent to give a compound of formula 3

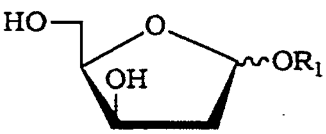

(3)

wherein $R_1$ is an alkyl group having 1 to 4 carbon atoms, after which position 5 is protected with a hydroxy protection group $R_4$ by methods known per se, or

b) if $R_4$ in formula 2 has the same meaning as $R_3$, with 1-1.5 equivalents of a nucleophilic carboxylate or benzylate to give a compound of formula 4

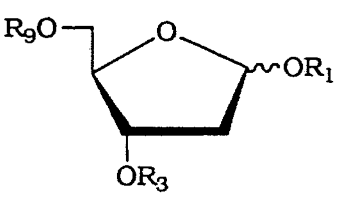

(4)

wherein $R_1$ and $R_3$ retain their meanings and $R_9$ is an acyl or benzyl group, after which at least a molar amount of an ionogenic nitrite in a dilution solvent is added, giving rise to a compound of formula 5

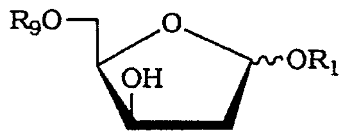

(5)

wherein $R_1$ and $R_9$ retain their meanings, or

c) if $R_4$ in formula 2 has the same meaning as $R_3$, with at least two equivalents of a nucleophilic carboxylate to give a compound of formula 6

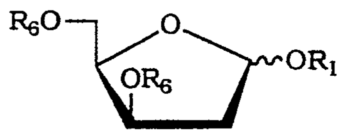

(6)

wherein $R_1$ retains its meaning and $R_6$ is an acyl group, after which $R_6$ is cleaved in the usual way and then position 5 is protected by a hydroxy protection group $R_4$ by methods known per se, or

d) if $R_4$ in formula 2 is a hydroxy protection group, with at least an equimolar amount of a nucleophilic carboxylate or an ionogenic nitrite in a dilution solvent to give a compound of formula 7

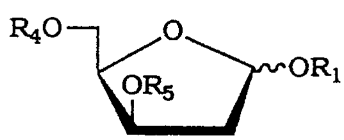

(7)

wherein $R_1$ and $R_4$ retain their meanings and $R_5$ is a hydrogen atom or an acyl group, after which $R_5$ is cleaved in the usual way if it is not already hydrogen.

In this process preferably a compound of the formula 2 is used wherein the hydroxy protection group $R_4$ is an alkanoyl, aroyl, 4-phenylaroyl, alkyloxycarbonyl, aryloxycarbonyl, aralkyl, alkyl-, aryl- or alkylaryl-silyl, tetrahydrofuranyl or tetrahydropyranyl group, which groups are either unsubstituted or substituted, preferably R4 is an triphenyl methyl, pivaloyl, isobutyloxycarbonyl, tetrahydropyranyl, 4-phenylbenzoyl or benzoyl group.

As said ionogenic nitrite compound preferably an alkali or tetraalkyl or alkaryl ammonium nitrite

having from 1 to 16 carbon atoms, preferably sodium nitrite, potassium nitrite or tetrabutylammonium nitrite is used.

A further subject matter of the present invention is a process for the manufacture of 3-substituted 2-deoxy-D-erythro-pentofuranose derivatives of formula 8

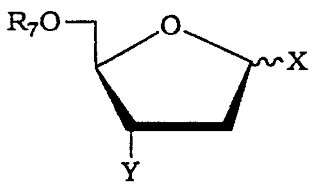

(8)

wherein $R_7$ is hydrogen or a hydroxy protection group, X is halogen or a group $OR_{10}$ wherein $R_{10}$ is hydrogen, an alkyl group having 1 to 4 carbon atoms or an acyl group and Y is halogen, an alkylthio, azido, cyano or thiocyanato group, which is characterized in that a 2-deoxy-D-threo-pentofuranoside of formula 1

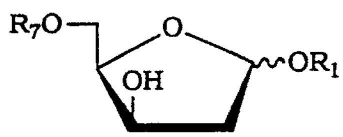

(1)

wherein $R_1$ is an alkyl group having 1 to 4 carbon atoms and $R_7$ is a hydroxy protection group, as obtained with the process according to claims 1 to 3, is converted by the addition of at least 1 mole of an alkyl-, aryl-, alkylaryl- or aralkyl-sulfonic acid halide or anhydride, either unsubstituted or substituted one or more times by halogen atoms, nitro or alkoxy groups, in the presence of a base or by addition of a 1-2 molar amount of sodium hydride followed by N,N'-sulfuryl-diimidazole or by reaction with an at least molar amount of sulfuryl chloride in the presence of imidazole or in the presence of a tertiary amine such as triethylamine, diethylarylamine, dimethylbenzylamine, pyridine, 2,6-dimethylamine with or without a dilution solvent that is inert under the reaction conditions such as pentane, hexane, benzene, toluene, xylene, diethylether, diisopropylether, petroleum fractions, dioxane, tetrahydrofurane, dichloromethane, chloroform, ethyl acetate or combinations thereof to a compound of formula 9

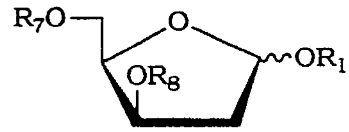

(9)

wherein $R_1$ and $R_7$ retain their meanings and $R_8$ is an alkyl-, aryl-, alkylaryl- or aralkyl-sulfonyl group that is either unsubstituted or substituted one or more times by halogen atoms, nitro or alkoxy groups, or signifies an imidazolesulfonyl or halosulfonyl group, after which the compound of formula 9 is converted by the addition of at least a molar amount of an ionogenic halide, alkylthiolate, azide, cyanide or thiocyanate compound or by reaction of the compound of formula 1 with at least an equivalent amount of a sulfurtrifluoride of a secondary amine such as dimethylamine, diethylamine or morpholine in a dilution solvent that is inert under the reaction conditions such as dichloromethane, chloroform, fluorotrichloromethane, toluene, benzene, xylene, tetrahydrofurane, dioxane, petroleum fractions, pentane, hexane, iso-octane, diethylether, to a compound of formula 10

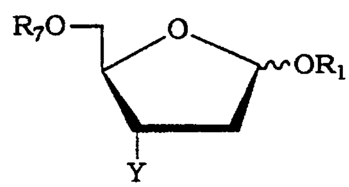

(10)

wherein $R_1$, $R_7$ and Y retain their meanings, whereafter said compound of formula 10, is transformed into a compound of formula 11 by methods known per se,

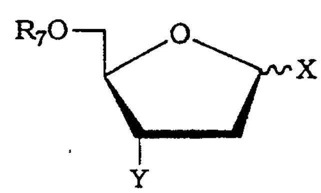

(11)

wherein Y and $R_7$ are as defined above, and X is $OR_{10}$ wherein $R_{10}$ is an acyl group or hydrogen, and,

if desired, said compound of formula 11 wherein X is $OR_{10}$ is reacted by methods known per se to provide a compound of formula 11 wherein Y and $R_7$ are as defined above, and X is halogen.

According to a preferred embodiment of this process a compound of formula 1 is used wherein the hydroxy protection group $R_7$ is an alkanoyl,

aroyl, alkyloxycarbonyl, aryloxycarbonyl, aralkyl, alkyl-, aryl- or alkylaryl-silyl, tetrahydrofuranyl or tetrahydropyranyl group, which groups are either unsubstituted or substituted, and that for the manufacture of the compounds of formula 11 a compound of formula 10 is used wherein the hydroxy protection group $R_7$ is an alkanoyl, aroyl, alkyloxycarbonyl or aryloxycarbonyl group, which groups are either unsubstituted or substituted, or the benzyl group, and $R_7$ preferably is an acetyl, benzyl, pivaloyl, isobutyloxycarbonyl or benzoyl group.

According to a further preferred embodiment of the present invention the 2-deoxy-D-erythro-pentofuranoside of formula 2 is manufactured by converting a 2-deoxy-D-erythro-pentose of the formula 12

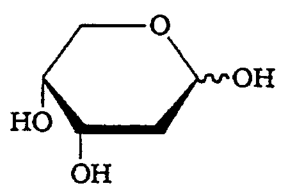

(12)

to a glycoside using an alkanol of formula $R_1OH$, wherein $R_1$ is an alkyl group having 1 to 4 carbon atoms to provide an alkyl 2-deoxy-D-erythropentofuranoside of formula 13

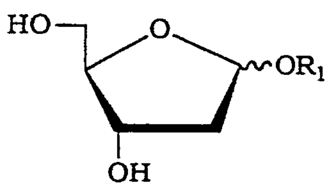

(13)

wherein $R_1$ is an alkyl group having 1 to 4 carbon atoms, then protecting position 5 by a hydroxy protection group $R_4$ by methods known per se to provide an alkyl 2-deoxy-D-erythro-pentofuranoside of formula 14

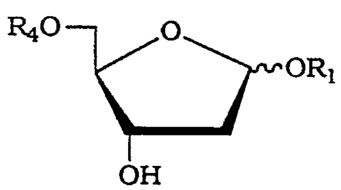

(14)

wherein $R_1$ is as defined above and $R_4$ is a hydroxy protection group, and then sulfonating said compound of formula 14 with 1-1.5 equivalents of an alkyl-, aryl-, alkylaryl- or aralkyl-sulfonic acid halide or anhydride, either unsubstituted or substituted one or more times by halogen atoms, nitro or alkoxy groups, in the presence of a base or by addition of a 1-2 molar amount of sodium hydride followed by N,N'-sulfuryl-diimidazole or by reaction with an at least molar amount of sulfuryl chloride in the presence of imidazole or in the presence of a tertiary amine such as triethylamine, diethylarylamine, dimethylbenzylamine, pyridine or 2,6-dimethylamine with a dilution solvent that is inert under the reaction conditions such as pentane, hexane, benzene, toluene, xylene, diethylether, diisopropylether, petroleum fractions, dioxane, tetrahydrofurane, dichloromethane, chloroform, ethyl acetate or combinations thereof.

In this process said position 5 of the pentofuranoside is protected with an alkanoyl, aroyl, 4-phenylaroyl, alkyloxycarbonyl, aryloxycarbonyl, aralkyl, alkyl-, aryl- or alkylaryl-silyl, tetrahydrofuranyl or tetrahydropyranyl group, which groups are either unsubstituted or substituted, and is preferably protected with an acetyl, pivaloyl, isobutyloxycarbonyl, tetrahydropyranyl, 4-phenylbenzoyl, 4-methyl-benzoyl or benzoyl group.

Here and in the following the alkyl groups comprise 1 to 10 carbon atoms, preferably 1 to 6 carbon atoms and most preferably are methyl, ethyl, propyl or butyl groups including the isomers thereof. The aryl groups preferably comprise phenyl or naphthyl groups. In the alkylaryl and aralkyl groups the alkyl and aryl residues correspond to the above definitions. Preferred aralkyl groups are the benzyl- and the triphenylmethyl group. The definition of halogen comprises fluorine, chlorine, bromine and iodine. Alkoxy substituent may comprise 1 to 10, preferably 1 to 6 carbon atoms, more preferably 1 to 4 carbon atoms, wherein the most preferable alkoxy group is the methoxy group. The acyl groups preferably are derived from aliphatic or aromatic carboxylic acids comprising 1 to 10 carbon atoms, more preferably 1 to 6 carbon atoms and most preferably 1 to 4 carbon atoms in the alkyl residue and comprising the phenyl residue in the aryl parts thereof. Specifically preferred acyl groups are acetyl, benzoyl, 4-methyl benzoyl, 4-phenylbenzoyl and pivaloyl groups.

Specifically preferred alkyl-, aryl-, alkylaryl- or aralkyl-sulfonic acid halides or anhydrides are methanesulfonyl chloride, p-toluene sulfonyl chloride, 4-bromobenzenesulfonyl chloride and trifluoromethane sulfonylchloride and trifluoromethane sulfonic acid anhydride.

Subject matter of the present invention furtheron are the intermediates prepared by and used in the processes of the invention and specifically:

A) 2-Deoxy-D-pentofuranose derivatives of the general formula 15

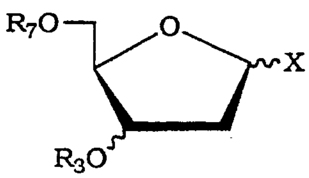

(15)

wherein $R_7$ is an alkanoyl, aroyl, alkyloxycarbonyl, aryloxycarbonyl, aralkyl, alkyl-, aryl- or alkylarylsilyl, tetrahydrofuranyl or tetrahydropyranyl group, which groups are either unsubstituted or substituted, preferably a a pivaloyl, tert.-butyldimethylsilyl, isobutyloxycarbonyl or tetrahydropyranyl group and $R_3$ is hydrogen or an alkyl-, aryl-, alkylaryl or aralkylsulfonyl group, either unsubstituted or substituted one or more times by halogen atoms, nitro or alkoxy groups, or an imidazolesulfonyl or halosulfonyl group, and X is a halogen atom or $OR_1$ wherein $R_1$ is an alkyl group having 1 to 4 carbon atoms or an acyl group, the threo and erythro derivatives and the α- and β-anomers thereof, with the exclusion of

methyl 2-deoxy-5-O-triphenylmethyl-D-erythro-pentofuranoside α and β anomer,

methyl 5-O-benzoyl-2-deoxy-D-erythro-pentofuranoside α/β mixture,

methyl 5-O-tert.butyldimethylsilyl-2-deoxy-D-erythro-pentofuranoside α/β mixture,

methyl 2-deoxy-3-O-methanesulfonyl-5-O-pivaloyl-D-erythro-pentofuranoside α anomer,

methyl 2-deoxy-5-O-(4-phenylbenzoyl)-D-erythro-pentofuranoside α and β anomer,

methyl 2-deoxy-5-O-triphenylmethyl-D-threo-pentofuranoside α and β anomer,

methyl 2-deoxy-5-O-(4-methylbenzoyl)-D-threo-pentofuranoside α/β mixture,

methyl 5-O-tert.butyldimethylsilyl-2-deoxy-D-threo-pentofuranoside β anomer,

methyl 2-deoxy-5-O-(4-phenylbenzoyl)-D-threo-pentofuranoside β anomer,

methyl 5-O-tert.butyldiphenylsilyl-2-deoxy-D-threo-pentofuranoside α and β anomer,

methyl 5-O-tert.butyldiphenylsilyl-2-deoxy-3-O-trifluoromethanesulfonyl-D-threo-pentofuranoside α and β anomer,

methyl 2-deoxy-D-threo-pentofuranoside α/β mixture.

B) 2-Deoxy-D-erythro-pentofuranosides of the general formula 2

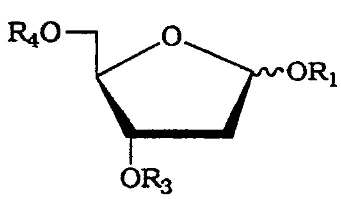

(2)

wherein $R_4$ is an alkanoyl, aroyl, alkyloxycarbonyl, aryloxycarbonyl, aralkyl, alkyl-, aryl- or alkylarylsilyl, tetrahydrofuranyl or tetrahydropyranyl group, which groups are either unsubstituted or substituted, preferably a pivaloyl, benzyl, 4-methylbenzoyl, tert.butyldimethylsilyl, isobutyloxycarbonyl or tetrahydropyranyl group. $R_3$ is hydrogen or an alkyl-, aryl-, alkylaryl- or aralkyl-sulfonyl group, either unsubstituted or substituted one or more times by halogen atoms, nitro or alkoxy groups, or an imidazolesulfonyl or halosulfonyl group, and $R_1$ is an alkyl group having 1 to 4 carbon atoms and the α- and β-anomers thereof, with the exclusion of

methyl 2-deoxy-5-O-triphenylmethyl-D-erythro-pentofuranoside α and β anomer,

methyl 5-O-benzoyl-2-deoxy-D-erythro-pentofuranoside α/β mixture,

methyl 5-O-tert.butyldimethylsilyl-2-deoxy-D-erythro-pentofuranoside α/β mixture,

methyl 2-deoxy-3-O-methanesulfonyl-5-O-pivaloyl-D-erythro-pentofuranoside α anomer,

methyl 2-deoxy-5-O-(4-phenylbenzoyl)-D-erythro-pentofuranoside α and β anomer,

Further preferred compounds are those of the above formula2 wherein $R_1$ and $R_4$ are as defined above and $R_3$ is an alkyl-, aryl-, alkylaryl- or aralkyl sulfonyl group, either unsubstituted or substituted one or more times by halogen atoms, nitro or alkoxy groups.

C) 2-Deoxy-D-threo-pentofuranosides of the general formula 9

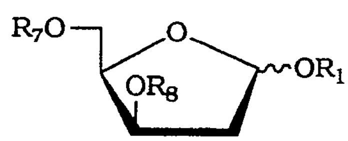

(9)

wherein $R_7$ is an alkanoyl, aroyl, alkyloxycarbonyl, aryloxycarbonyl, aralkyl, alkyl-, aryl- or alkylarylsilyl, tetrahydrofuranyl or tetrahydropyranyl group, which groups are either unsubstituted or substituted, preferably a pivaloyl, benzyl, tert.-butyldimethylsilyl, isobutyloxycarbonyl or tetrahydropyranyl group, $R_8$ is hydrogen or an alkyl-, aryl-, alkylaryl- or aralkylsulfonyl group, either unsubstituted or substituted one or more times by halogen atoms, nitro or alkoxy groups, a benzoyl or acetyl group, or an imidazolesulfonyl or halosulfonyl group, and $R_1$ is an alkyl group having 1 to 4 carbon atoms, and the α- and β-anomers thereof, with the exclusion of

methyl 2-deoxy-5-O-triphenylmethyl-D-threo-pentofuranoside α and β anomer,

methyl 2-deoxy-5-O-(4-methylbenzoyl)-D-threo-pentofuranoside α/β mixture,
methyl 5-O-tert.butyldimethylsilyl-2-deoxy-D-threo-pentofuranoside β anomer,
methyl 2-deoxy-5-O-(4-phenylbenzoyl)-D-threo-pentofuranoside β anomer,
methyl 5-O-tert.butyldiphenylsilyl-2-deoxy-D-threo-pentofuranoside α and β anomer,
methyl 5-O-tert.butyldiphenylsilyl-2-deoxy-3-O-trifluoromethanesulfonyl-D-threo-pentofuranoside α and β anomer,
methyl 2-deoxy-D-threo-pentofuranoside α/β mixture,
methyl 3-O-acetyl-5-O-benzyl-2-deoxy-D-threo-pentofuranoside α/β anomer mixture.

Subject matter of the present invention furtheron are compounds of the above general formula 9, wherein $R_7$ is benzoyl 4-phenylbenzoyl, pivaloyl or tert.butyldimethylsilyl and $R_8$ is an alkyl-, aryl-, alkylaryl- or aralkyl sulfonyl group, either unsubstituted or substituted one or more times by halogen atoms, nitro or alkoxy groups.

D) 2-Deoxy-D-erythro-pentofuranose derivatives of the general formula 10

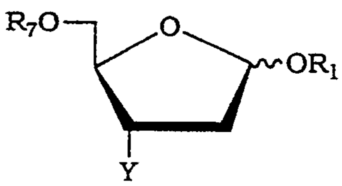

(10)

wherein $R_7$ is a pivaloyl, triphenylmethyl, tert.-butyldimethylsilyl, isobutyloxycarbonyl or tetrahydropyranyl group, Y is halogen, an azido, cyano or thiocyanato group and $R_1$ is an alkyl group having 1 to 4 carbon atoms, and the α- and β-anomers thereof.

E) 2-Deoxy-D-erythro-pentofuranose derivatives of the general formula 11

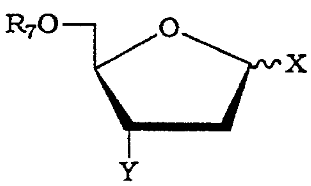

(11)

wherein $R_7$ is a pivaloyl, triphenylmethyl, tert.-butyldimethylsilyl, isobutyloxycarbonyl or tetrahydropyranyl group, Y is halogen, an azido, cyano or thiocyanato group and X is halogen or a group $OR_{10}$ wherein $R_{10}$ is hydrogen or an acyl group, and the α- and β-anomers thereof.

Specifically preferred compounds according to the present invention are methyl 2-deoxy-5-O-pivaloyl-D-erythro-pentofuranoside, methyl 2-deoxy-3-O-methanesulfonyl-5-O-pivaloyl-D-erythro-pentofuranoside, methyl 2-deoxy-5-O-pivaloyl-D-threo-pentofuranoside, methyl 2,3-dideoxy-3-fluoro-5-O-pivaloyl-D-erythro-pentofuranoside and the α- and β-anomers thereof and 2,3-Dideoxy-3-fluoro-D-erythro-pentofuranose. Subject matter of the present invention furtheron is the use of the compounds of formula 10 or 11 as defined above with the exception of a compound of formula 11 wherein X is OH, for the manufacture of 2'-deoxynucleosides of the following general formula 16

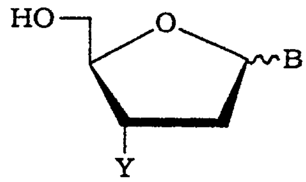

(16)

wherein Y is a halogen, an azido, cyano or thiocyanato group and B is a N-heterocyclic base residue, preferably a purine or pyrimidine base residue, which is characterized in that a compound of said formula 10 or 11, wherein X is halogen or a group $OR_{10}$, wherein $R_{10}$ is an acyl group, is condensed with a N-heterocyclic base, preferably with a purine or pyrimidine base, using methods known per se, whereafter any protective groups present are cleaved off and, if desired, the anomers of the compounds of formula 16 are separated by crystallisation or chromatography using methods known per se. Preferably compounds of the above formula 10 are used wherein $R_1$ is a methyl group and $R_7$ is an acyl group, which are condensed with at least an equimolar amount of a silylated purine or pyrimidine base under catalysis, the protective groups present are cleaved off and the anomers of formula 16 formed are separated by crystallization or chromatography using methods known per se.

The starting material for performing the process according to the invention can be the mixture of alkyl 2-deoxy-D-erythro-pentofuranosides obtainable from readily accessible 2-deoxy-D-erythro-pentose (2-deoxy-D-ribose) (formula 12) by simple Fischer glycosidation, e.g. according to R.K. Ness, (J. Org. Chem., 1961, 26, 2895-2899). These compounds can be protected by a hydroxy protection group in position 5 in the usual way before use to provide the compounds of formula 14.

The following compounds are particularly used as agents supplying protective groups:

A) alkyl or aryl carboxylic acid halides or anhydrides, alkoxy or aryloxycarbonyl halides as well

as triphenylmethyl halides, which are used in an equivalent or slightly excess amount relative to the numbers of hydroxy groups to be protected and in the presence of at least an equivalent amount of a base, e.g. an organic base such as pyridine, triethylamine, a dilution solvent that is inert under the reaction conditions, such as a hydrocarbon, e.g. benzene, toluene, petroleum fractions, or a chlorinated hydrocarbon such as dichloromethane, chloroform, carbon tetrachloride, an ether such as diethylether, diisopropyl ether, or an inorganic base such as an alkali hydroxide, alkali bicarbonate or alkali carbonate, in a solvent mixture containing water, e.g. water/acetone, water/toluene, at temperatures of about -20 to 100 °C preferably from about 0 to 25 °C, whereby acyl groups such as alkanoyl, aroyl or 4-phenylbenzene groups, alkyloxycarbonyl, aryloxycarbonyl or triphenylmethyl groups, which groups are either unsubstituted or substituted, are introduced as said hydroxy protection group,

B) alkyl or aralkyl halides or sulfonates, preferably benzyl derivatives whereby a salt is first formed with the hydroxy group to be protected, for example by adding at least one equivalent of an alkali metal hydride in a dilution solvent that is inert under the reaction conditions, such as an N,N-dialkylcarboxylic acid amide, e.g. N,N-dimethylformamide, or a cyclic ether such as tetrahydrofuran or mixtures thereof at temperatures from about -50 to 50 °C, after which the reaction is performed by adding at least one equivalent of one of the aforesaid compounds in a dilution solvent that is inert under the reaction conditions at temperatures of about -20 to 50 °C, whereby alkyl or aralkyl groups are introduced as the hydroxy protection group,

C) cyclic vinyl ethers, preferably 3,4-dihydro-2H-pyran, wherein the reaction is performed in a dilution solvent that is inert under the reaction conditions, such as halogenated hydrocarbons, e.g. dichloromethane, in the presence of a catalyst, e.g. pyridinium toluenesulfonate at temperatures from about -20 to 50 °C, whereby the tetrahydropyranyl group is introduced as the hydroxy protection group,

D) trialkyl-, aryl-dialkyl- or diaryl-alkyl-silylhalides, wherein the reaction is performed in a dilution solvent such as N,N-dimethylformamide for example in the presence of at least an equimolar amount of a base such as imidazole at temperatures from about -20 to 25 °C, whereby the trialkyl-, aryl-dialkyl- or diaryl-alkyl-silyl groups are introduced.

In formula 14, $R_1$ preferably means a methyl group and $R_4$ hydroxy protection group which is stable under the conditions of the introduction of the azido, cyano, thiocyanato group or the halogen. Examples of such protective groups are listed under (A) to (C).

If a 3'-substituted-2'-deoxy-nucleoside is to be manufactured, preferred compounds for use as starting products are those in which $R_4$ is not cleaved during the course of condensation with purine or pyrimidine bases, e.g. according to US-A-4,082,911, for example an acyl group such as acetyl, benzoyl or pivaloyl group, wherein the pivaloyl group is particularly preferred. The mixture of methyl 2-deoxy-5-O-pivaloyl-D-erythro-pentofuranoside is quite particularly preferred as the starting product of formula 14. These compounds can be manufactured by the addition of at least of one equivalent of pivalic acid chloride to a dichloromethane solution of methyl 2-deoxy-D-erythro-pentafuranoside of formula 13 at temperatures of about -20 to 25 °C, stirring until the reaction is completed, shaking with dilute hydrochloric acid solution and water, drying over sodium sulphate and evaporating the solvent, whereby methyl 2-deoxy-5-O-pivaloyl-alpha,beta-D-erythro-pentafuranoside arises and can be purified further by chromatography or, preferably, be used without further purification.

To perform the process according to the invention, the compound of formula 14 wherein $R_1$ is an alkyl group with 1 to 4 carbon atoms and $R_4$ is a hydroxy protection group, is taken up in a dilution solvent that is inert under the reaction conditions, such as a hydrocarbon or a chlorinated hydrocarbon, preferably a chlorinated hydrocarbon, and is mixed with at least one equivalent of a base per hydroxy group. Organic bases such as pyridine, triethylamine, preferably triethylamine, come into question as bases, whereby the base alone can be used as the dilution solvent. It is preferred to use 1-1.5 equivalents of base per hydroxy group to be protected.

This is then reacted by stirring with an alkyl-, aryl-, alkylaryl- or aralkylsulfonic acid halide or anhydride, either unsubstituted or substituted one or more times by halogen atoms, nitro or alkoxy groups with 1 to 5 carbon atoms, preferably methanesulfonic acid chloride, nitro, 4-bromobenzene sulfonic acid chloride or toluenesulfonic acid chloride. The sulfonic halide or anhydride may be added as such or in one of the dilution solvents stated above. It is added in an equimolar amount or in excess, preferably about 1.1 equivalents per hydroxy group. The reaction temperature thereby is about from -20° to the boiling point of the dilution solvent used. Depending on the starting material used, this gives rise to a compound of formula 2 wherein $R_1$ is an alkyl group having 1 to 4 carbon atoms, $R_3$ is an alkyl, aryl, alkylaryl or aralkylsulfonyl group which is unsubstituted or substituted one or more times by halogen atoms, nitro or alkoxy groups, and $R_4$ has the meaning of $R_3$ if $R_4$ meant hydrogen before sulfonylation, or retains its meaning if $R_4$ was a hydroxy protection group before sulfonylation.

The compound of formula 2 arising in this reaction is either processed in the usual way, e.g. by adding water and extracting with aqueous solutions of $KHSO_4$ and $NaHCO_3$ and water, drying and evaporating the dilution solvent and purified by crystallization and recrystallization or column chromatography or is used further without purification.

A Walden inversion is performed in the next process step with substitution of the sulfonic acid group in position 3. This reaction is performed in excess O-nucleophilic material such as carboxylate, particularly alkali- or tetraalkylammonium benzoate or acetate, mono-, di- or trihaloacetate, whereby potassium acetate or sodium benzoate are particularly preferred, or ionogenic nitrites such as alkali or tetraalkylammonium nitrite, preferably potassium or sodium nitrite, in dilution solvent such as aprotic dipolar dilution solvents, e.g. acetonitrile, N,N-dialkylcarboxylic acid amides, such as N,N-dimethyl formamide, N,N-dimethylpropylene urea (DMPU), dimethylsulphoxide, with or without additional solvent such as tetrahydrofuran or dioxan, at temperatures between about -30 and 200° C.

If a carboxylate is used as the O-nucleophilic material, the sulfonic acid groups introduced in the previous step are replaced by the corresponding acyloxy groups; if an ionogenic nitrite is used as O-nucleophilic material, the compound with free hydroxy groups is isolated. In many cases it may be advantageous to use a carboxylate as O-nucleophilic material since this gives rise to compounds which can be purified well by simple distillation.

By substitution of the sulfonic acid groups, the following compounds arise from the compound of formula 2:

a) if $R_4$ in formula 2 has the same meaning as $R_3$, the addition of at least two equivalents of ionogenic nitrite in a dilution solvent leads to a compound of formula 3

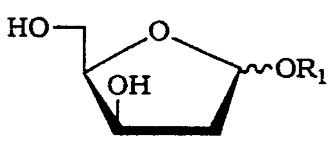

(3)

wherein $R_1$ is an alkyl group having 1 to 4 carbon atoms, after which position 5 is protected with a hydroxy protection group as described under (A) - (C),

b) if $R_4$ in formula 2 has the same meaning as $R_3$, the addition of 1-1.5 equivalents of a nucleophilic carboxylate leads to a compound of formula 4

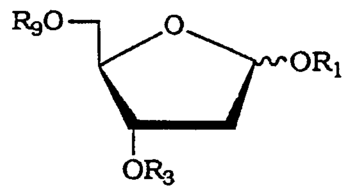

(4)

wherein $R_1$ and $R_3$ retain their meanings and $R_9$ is an acyl group, after which at least a molar amount of an ionogenic nitrite in a dilution solvent is added, giving rise to a compound of formula 5

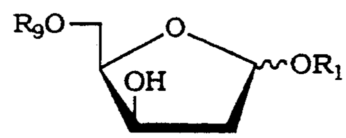

(5)

wherein $R_1$ and $R_9$ retain their meanings,

c) if $R_4$ in formula 2 has the same meaning as $R_3$, the addition of at least two equivalents of a carboxylate gives rise to a compound of formula 6

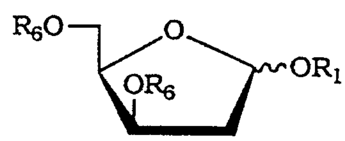

(6)

wherein $R_1$ retains its meaning and $R_6$ is an acyl group, after which $R_6$ is cleaved in the usual way and after which position 5 is protected by a hydroxy protection group as described above under (A) to (D),

d) if $R_4$ in formula 2 is a hydroxy protection group, the addition of at least an equimolar amount of a carboxylate or nitrite in a dilution solvent gives rise to a compound of formula 7

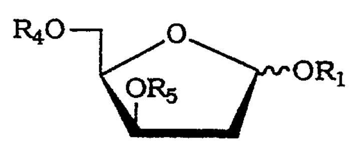

(7)

wherein $R_1$ and $R_4$ retain their meanings and $R_5$ is a hydrogen atom or an acyl group, after which $R_5$ is cleaved in the usual way if it is not already hydrogen,

whereby in all cases (a) to (d) a compound of formula 1

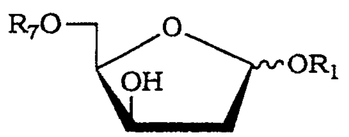

(1)

arises wherein $R_1$ is an alkyl group having 1 to 4 carbon atoms and $R_7$ is a hydroxy protection group.

If $R_1$ in formula 3 is cleaved in the usual way, 2-deoxy-D-threo-pentose arises.

In the next process step, sulfonylation is again undertaken in position 3 under the conditions described above when using the previously described sulfonic acid halides or anhydrides or an imidazolesulfonyl or halosulfonyl group is introduced, whereby a compound of formula 9

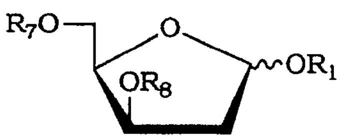

(9)

arises, wherein $R_1$ and $R_7$ retain their meanings and $R_8$ is an alkyl-, aryl-, alkylaryl- or aralkylsulfonyl group that is unsubstituted or substituted one or more times by halogen atoms or alkoxy groups, the imidazole sulfonyl or the halosulfonyl group. A trifluoromethanesulfonyl group, imidazolesulfonyl group or methane sulfonyl group are preferably introduced.

The manufacture of compounds in which $R_8$ is a trifluoromethanesulfonyl group is undertaken by dissolving the compound of formula 1 in a dilution solvent that is inert under the reaction conditions, such as chlorinated hydrocarbon, e.g. dichloromethane, and adding an approximately equimolar amount of trifluoromethane sulfonic anhydride in the presence of a base such as pyridine with stirring at temperatures from about -50 to 25° C. The manufacture of compounds in which $R_8$ is an imidazolesulfonyl group is undertaken for example by dissolving a compound of formula 1 in a dilution solvent that is inert under the reaction conditions, such as N,N-dimethylformamide, adding at least an equimolar amount of sodium hydride followed by an approximately equimolar amount of N,N-sulfuryl-diimidazole or the addition of at least an equimolar amount of sulfurylchloride followed by an excess of imidazole at temperatures from about -50 to 20° C.

After evaporation of the dilution solvent if necessary, the compound of formula 9 is taken up in a solvent not miscible with water, preferably dichloromethane, and the mixture is extracted with aqueous solutions of, for example, $KHSO_4$ and $NaHCO_3$, and with water. The organic phase is dried and the solvent evaporated, after which the residue may be purified by crystallization or chromatography. It is preferably not to perform purification.

The compound of formula 9 is converted by Walden inversion into a compound of formula 10

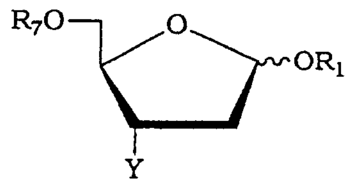

(10)

wherein $R_1$ and $R_7$ retain their meanings and Y is a fluoro, azido, cyano or thiocyanate group.

The introduction of the azido group is performed by adding at least an equimolar amount of an ionogenic azide such as potassium, sodium or a caesium or lithium azide in a dilution solvent that is inert under the reaction conditions, such as a ketone, e.g. diphenyl ketone, diethyl ketone, an ether, such as tetrahydrofuran, a nitrile such as acetonitrile, a carboxylic acid amide such as N,N-dimethylacetamide, N,N-dimethylformamide; dimethylsulfoxide, ethyleneglycol, sulfolan, ethyleneglycol ether, DMPU or mixtures of solvents suitable for $S_N2$ reactions at temperatures of about -30 to 200° C, preferably 50 to 200° C. After the end of the reaction, the dilution agent is distilled off if the dilution agent is miscible with water, and the residue is taken up in an organic dilution solvent not miscible with water and water, or the reaction mixture is instantly distributed between the organic dilution solvent not miscible with water and water. The organic phase is then dried and evaporated, after which purification may be undertaken by crystallization and recrystallization or by chromotography.

The protective groups in position 1 and position 5 can be cleaved from the compound of formula 10 in the usual way. Depending on their nature, the hyroxy protection groups $R_1$ and $R_7$ may be cleaved at the same time or in sequence.

By cleavage of $R_1$ and $R_7$, 3-azido-2,3-dideoxy-D-erythro-pentose is obtained. This compound is new and also an object of the invention.

The introduction of the fluoro group is provided by addition of an at least equimolar amount of an ionic fluoride such as potassium, sodium or caesium fluoride, $KHF_2$ or quarternery ammonium fluoride.The reaction is carried out in a dilution

solvent that is inert under the reaction conditions as defined above, whereafter the product is isolated and purified as described above whereafter the protective groups may be split off by methods known per se. For example, the hydroxy protecting groups $R_1$ and $R_7$ can be split off simultaneously or optionally in succession. 3-Fluoro-2,3-dideoxy-D-erythro-pentose is produced by splitting off hydroxy protecting groups $R_1$ and $R_7$. This compound is novel and is likewise an object of the invention.

For the manufacture of compounds of formula 8, wherein Y is a cyano or thiacyanate group, the reaction is carried out in a similar way making use of an ionic cyanide or thiocyanate compound, such as potassium, sodium or caesium cyanide or thiocyanate, preferably a tetraalkylammonium cyanide or thiocyanate, such as tetrabutylammonium cyanide or thiocyanate.

Another further object of the invention is the use of the compounds of formula 10 manufactured in this way for the preparation of 3'-substituted-2'-deoxy-nucleosides by condensation with N-heterocyclic bases protected in suitable ways.
Subject matter of the present invention therefore is the use of compounds of formulae 10 or 11 with the exception of a compound of formula 11 wherein X is OH for the manufacture of a nucleoside of formula 16

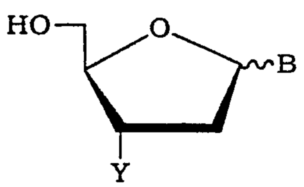
(16)

wherein Y has the meaning stated above and B is the residue of a N-heterocyclic base, comprising the condensation of a N-heterocyclic base with said compounds by a method suitable for condensation with N-heterocyclic bases. The condensation is preferably performed according to US-A-4,082,911, whereby $R_1$ in formula 10 is preferably a methyl group and $R_7$ is preferably a pivaloyl group. Purine or pyrimidine bases in which the ring may also exhibit the desired modifications can be considered as purine or pyrimidine bases, whereby each position in the ring which is reactive under condensation conditions must be protected by protective groups.

For example, to form the nucleoside containing the pyrimidine base, a compound of formula 10 is reacted with at least an equimolar amount of 2,4-bis-(trimethylsilyloxy)-pyrimidine in a dilution solvent that is inert under the reaction conditions such as acetonitrile, and in the presence of a catalyst such as trimethylsilyl trifluoromethane sulfonate for example at temperatures of -20 to 40 °C. The formation of nucleosides containing purine bases can also be undertaken in the manner described above, e.g. using 6-benzoyltrimethylsilyl-amino-9-trimethylsilylpurine as the starting compound. The compounds obtained in this way may be purified by chromatography or by crystallization for example.

Cleavage of the hydroxy protection group $R_7$ from the compounds obtained are performed in a manner suitable for the protective group in question. If $R_7$ is an alkoxycarbonyl, aryloxycarbonyl or acyl group, cleavage is preferably performed by the addition of equimolar amounts of sodium methylate in methanol, stirring at room temperature and neutralizing, preferably by means of an ion-exchanger. Isolation may be performed by evaporating the dilution solvent or precipitating the compound by adding chloroform, and purification may be performed by crystallization and recrystallization or by chromatography.

The reaction gives rise to a mixture of anomeric compounds. Separation of the anomers can be performed by chromatography or crystallization and is suitably applied to the end product.

In a preferred embodiment of the process, 2-deoxy-D-ribose is first protected in position 1 by conversion to the methyl glycoside. An acyl group is then introduced into position 5 in the usual way, after which position 3 is sulfonylated with the aid of methane sulfonic acid chloride as described above. The sulfonic acid group $R_3$ is then substituted by a hydroxy group with the aid of sodium nitrite as described above. According to another preferred embodiment, 2-deoxy-D-ribose is glycosylated first with methanol and then sulfonylated with at least 2 molar amounts of methanesulfonic acid chloride as described above, whereby the sulfonylation occurs in positions 3 and 5. The sulfonic acid groups are substituted by hydroxy groups by the addition of sodium nitrite as described above, after which position 5 is protected as described above.

According to both preferred embodiments, a compound of formula 1 wherein $R_1$ is a methyl group and $R_7$ is an acyl group arises, which is preferably sulfonylated in position 3 with the aid of trifluoromethanesulfonic acid anhydride, methanesulfonic acid chloride, 4-bromobenzenesulfonic acid chloride or p-toluenesulfonic acid chloride as described above. By addingthe nucleophile such as azide, halide, cyanide or thiocyanate as described above, a compound of formula 10 arises from this, wherein $R_1$ is a methyl group, $R_7$ is an acyl group and Y is an azido, cyano, thiocyanato group or halogen. By the addition of a suitably protected purine or pyrimidine base, the compound of formula 10 can then be converted into the corresponding 3'-substituted-2'-deoxy nucleoside,

from which protective group $R_7$ can be cleaved for example by the addition of sodium methylate in methanol, after which the anomers may be chromatographically separated if desired.

The process provides good yields, can be performed on a large technical scale in a particularly economic manner using readily accessible starting substances, and thus represents an enrichment of the technique.

**EXAMPLES**

**Example 1:**

74 g (0.5 mole) of methyl 2-deoxy-D-erythro-pentofuranoside, manufactured according to Robert K. Ness, J. Org. Chem. 26(1961), 2895-2899, were dissolved in 750 ml of dichloromethane and 47.5 g (0.6 mole) of pyridine and 66.3 g (0.55 mole) of pivalic acid chloride dissolved in 150 ml dichloromethane were added dropwise slowly to this solution at about 5° C with vigorous stirring. The reaction solution was heated to room temperature and stirred for 24 hours. After cooling to 5° C, the solution was mixed with iced water and the excess pyridine removed by acidification with 10% aqueous hydrochloric acid and several washings with water. The organic phase was dried over sodium sulphate and evaporated in a rotary evaporator. This gave 106g (0.46 mole), i.e. 92% of theoretical, of **methyl 2-deoxy-5-O-pivaloyl-D-erythro-pentofuranoside**, which was used further without additional purification.

**alpha-anomer**
**$^1$H-NMR ($CDCl_3$)**
5.11 (H-1, J 4.5), 2.17 ($H-2_a$, J 4.5, 6.4, 13.8), 2.03 ($H-2_b$, J 13.8), 4.05-4.20 (H-3, $H-5_a$, $H-5_b$, m), 4.26 (H-4, J 2.1, 4.5, 4.5); 3.33 ($OCH_3$), 1.20(3 $CH_3$).
**$^{13}$C-NMR ($CDCl_3$)**
106.1 (C-1), 41.4 (C-2), 73.4 (C-3), 85.4 (C-4), 64.6 (C-5); 55.3 ($OCH_3$); 27.5/39.5/179.4 (OPiv)

**beta-anomer**
**$^1$H-NMR ($CDCl_3$)**
5.08 (H-1, J 1.7, 5.3), 2.26 ($H-2_a$, J 1.7, 6.7, 13.2), 2.09 ($H-2_b$, J 5.3, 6.7, 13.2), 4.41 (H-3, J 4.7, 6.7, 6.7), 4.05 (H-4, J 4.7, 5.3, 5.3), 4.17 ($H-5_a$, $H-5_b$,J 5.3); 3.40 ($OCH_3$), 1.22 ($3CH_3$)
**$^{13}$C-NMR ($CDCl_3$)**
105.8 (C-1), 41.9 (C-2), 72.9 (C-3), 84.4 (C-4), 65.5 (C-5); 55.3 ($OCH_3$); 27.5/39.6/179.9 (OPiv)

**Example 2:**

14.8 g (0.1 mole) of methyl 2-deoxy-D-erythro-pentofuranoside prepared as in Example 1 were dissolved in a mixture of 200 ml absolute dichloromethane and 20 g (0.25 mole) absolute pyridine, and mixed with 30.7 g (0.11 mole) chlortriphenylmethane with stirring. After standing overnight at room temperature, the mixture was heated to 40° for one hour and then cooled. The organic phase was then extracted with aqueous solutions of $KHSO_4$ and $NaHCO_3$, and then with water. After drying over $Na_2SO_4$, the solvent was evaporated and the residue chromatographically purified (ethyl acetate/toluene = 1/1). This gave 34.7 g (0.089 mole), i.e. 89% of theoretical, of **metyhl 2-deoxy-5-O-triphenylmethyl D-erythro-pentofuranoside**.

**alpha-anomer**
**$^1$H-NMR ($CDCl_3$)**
5.13 (H-1, J 4.5), 2.19 ($H-2_a$, J 4.5, 5.9, 13.5), 2.00-($H-2_b$, J 13.5), 4.17 (H-3, J 1.5, 5.9), 4.22 (H-4, J 1.5, 4.5, 4.5), 3.15 ($H-5_a$, $H-5_b$, J 4.5); 3.37 ($OCH_3$), 7.15 - 7.45(3 Ph)
**$^{13}$C-NMR ($CDCl_3$)**
106.3 (C-1), 41.4 (C-2), 74.0 (C-3), 87.3 (C-4), 64.8 (C-5); 55.4 ($OCH_3$); 87.8/128.0/128.9/129.5/144.8 (OTr)

**beta-anomer**
**$^1$H-NMR ($CDCl_3$)**
5.04 (H-1, J 2.1, 5.3), 2.13 ($H-2_a$, J 2.1, 6.5, 13.1), 2.00 ($H-2_b$, J 5.3, 6.5, 13.1), 4.35 (H-3, J 5.8, 6.5, 6.5), 3.96 (H-4, J 5.5, 5.8, 6.0), 3.29 ($H-5_a$, J 5.5, 9.6), 3.17 ($H-5_b$, J 6.0, 9.6); 3.26 ($OCH_3$), 7.2-7.5 (3 Ph)
**$^{13}$C-NMR ($CDCl_3$)**
105.8 (C-1), 41.6 (C-2), 73.6 (C-3), 85.4 (C-4), 65.8 (C-5); 55.6 ($OCH_3$); 87.5/128.0/128.9/129.5/144.9 (OTr)

**Example 3:**

1.48 g (10 mmol) of methyl 2-deoxy-D-erythro-pentofuranoside were dissolved in 20 ml of a mixture of dimethylformamide and tetrahydrofuran (1:1) and reacted with 0.3 g (12.5 mmol) of sodium hydride with stirring. After stirring for one hour, the mixture was cooled to 0° C and added dropwise to 1.4 g (11 mmol) benzyl chloride dissolved in 10 ml of tetrahydrofuran, and stirred for a further 12 hours at room temperature. Dichloromethane and water were then added to the reaction mixture. The organic phase was washed with an aqueous solution of $KHSO_4$ and $NaHCO_3$ and with water, dried over sodium sulphate and evaporated in a rotary evaporator. The oily residue was purified by column chromatography on silica gel (ethyl acetate/toluene = 5/1). This gave 1.69 g (7.1 mmol), i.e. 71% of theoretical, of **methyl 5-O-benzyl-2-deoxy-D-erythro-pentofuranoside**.

**alpha/beta-anomer mixture**
**$^{13}$H-NMR ($CDCl_3$)**
105.9/106.4 (C-1), 41.5/41.6 (C-2), 72.6/74.0 (C-3), 85.1/86.9 (C-4), 70.7/71.1 (C-5); 55.4/55.6 ($OCH_3$); 71.1/128.5/128.6/129.3/138.9 (OBn)

**Example 4:**

2.0 g (29.4 mmol) of imidazole were added with stirring to a solution of 1.48 g (10 mmol) of methyl 2-deoxy-D-erythro-pentofuranoside in 25 ml of N,N-di-methylformamide, after which the mixture was cooled to 4°C and a solution of 1.6g (10.6 mmol) of tert.butyldimethylchlorosilane in 5 ml DMF was added dropwise at this temperature. After one hour, the solvent was evaporated in a rotary evaporator. The residue was taken up in 30 ml dichloromethane and 15 ml water, and the organic phase was extracted with water, dried over sodium sulphate and evaporated. This gave 2.5 g (9.5 mmol), i.e. 95% of theoretical, of **methyl 5-O-tert.butyldimethylsilyl-2-deoxy-D-erythro-pentofuranoside**.

**alpha/beta-anomer mixture**
**$^{1}$H-NMR ($CDCl_3$)**
5.0-5.1 (H-1,m), 1.9-2.3 (H-$2_a$, H-$2_b$,m), 3.5-4.5 (H-3, H-4, H-$5_a$, H-$5_b$,m); 3.31/3.38 ($OCH_3$), 0.05/0.08/0.89/0.91 (OTBDMS)

**Example 5:**

58.0 g (0.25 mole) of methyl 2-deoxy-5-O-pivaloyl-α-D-erythro-pentofuranoside, prepared according to Example 1, were dissolved in 650 ml of dichloromethane and mixed with 38 g (0.375 mole) of triethylamine. The solution was cooled to 0°C and mixed dropwise with stirring with 37.2 g (0.325 mole) of methane sulfonic acid chloride dissolved in 70 ml dichloromethane. After about 30 minutes, the reaction mixture was poured into iced water and the organic phase extracted with an aqueous solution of $KHSO_4$ and $NaHCO_3$ and then with water then dried over sodium sulphate and evaporated in a rotary evaporator. This gave 72.3 g (0.23 mole), i.e. 93% of theoretical, of **methyl 2-deoxy-3-O-methanesulfonyl-5-O-pivaloyl-D-erythro-pentofuranoside**, which was used further without additional purification.

**alpha-anomer**
**$^{1}$H-NMR ($CDCl_3$)**
5.13 (H-1, J 5.1), 2.41 (H-$2_a$, J 5.1, 7.9, 14.9), 2.26 (H-$2_b$, J 2.0, 14.9), 5.09 (H-3, J 2.0, 3.5, 7.9), 4.43 (H-4, J 3.5, 3.9, 3.9), 4.33 (H-$5_a$, J 3.9, 12.0), 4.22 (H-$5_b$, J 3.9, 12.0); 3.09 (OMs), 3.40 ($OCH_3$), 1.22 (3 $CH_3$)
**$^{13}$C-NMR ($CDCl_3$)**
105.4 (C-1), 39.8 (C-2), 79.4 (C-3), 81.5 (C-4), 63.4 (C-5); 55.6 ($OCH_3$); 27.5/39.2/179.2 (OPiv); 39.0 (OMs)

**Example 6:**

Using 1.5 equivalents of p-toluene sulfonic acid chloride, **methyl 2- deoxy-5-O-pivaloyl-3-O-(p-toluenesulfonyl)-D-erythro-pentofuranoside** was prepared as described in Example 5, with a yield of 90% of theoretical.

**alpha/beta-anomer mixture**
**$^{13}$C-NMR ($CDCl_3$)**
105.1/104.6 (C-1), 39.3 (C-2), 79.6/80.4 (C-3), 80.8/81.3 (C-4), 62.8/63.7 (C-5); 55.3/55.0 ($OCH_3$); 27.2/39.3/179.5 (OPiv);
21.8/127.1/127.9/130.1/130.4/141.8 (OTs)

**Example 7:**

Using 1.2 equivalents of sulfuryl chloride, **methyl 2-deoxy-5-O-pivaloyl-3-O-chloro-sulfuryl-D-erythro-pentofuranoside** was prepared as described in Example 5, with the yield of 86% of theoretical.

**alpha/beta-anomer mixture**
**$^{13}$C-NMR ($CDCl_3$)**
105.8/105.2 (C-1), 39.2/39.1 (C-2), 87.7/88.9 (C-3), 80.8/81.2 (C-4), 63.3/63.8 (C-5); 55.7/55.5 ($OCH_3$); 27.4/39.4/179.5 (OPiv).

**Example 8:**

Using 1.5 equivalents of 4-bromobenzenesulfonyl chloride, **methyl 3-O-(4-bromo-benzenesulfonyl)-2-deoxy-5-O-pivaloyl-D-erythro-pentofuranoside** was prepared as described in Example 5 with a yield of 92% of theoretical.

**alpha-anomer**
**$^{1}$H-NMR ($CDCl_3$)**
5.05 (H-1, J 1.0, 5.2), 2.25 (H-2a, J 5.2, 7.9, 14.8), 2.07 (H-2b, J 1.0, 2.1 14.8), 4.91 (H-3, J 2.1, 3.6, 7.9), 4.38 (H-4, J 3.6, 3.6, 3.9), 4.24 (H-5a, J 3.6, 12.0), 4.10 (H-5b, J 3.9, 12.0); 3.35 ($OCH_3$); 1.19 (3 $CH_3$); 7.7-7.85 (OBBS).
**$^{13}$C-NMR ($CDCl_3$)**
105.3 (C-1), 39.5 (C-2), 80.8, 81.2 (C-3, C-4), 63.3 (C-5); 55.6 ($OCH_3$); 27.5/39.1/179.5 (OPiv); 130.2/133.6/133.7/136.5/ (OBBS)

**beta-anomer**
**$^{13}$C-NMR ($CDCl_3$)**
105.8 (C-1), 39.7 (C-2), 81.9 (C-3, C-4), 64.1 (C-5); 55.8 ($OCH_3$);
27.5/39.0/179.5 (OPiv); 130.4/133.6/133.7/136.4

(OBBS)

**Example 9:**

Starting from methyl 2-deoxy-5-O-triphenylmethyl D-erythro-pentofuranoside, **methyl 2-deoxy-3-O-methanesulfonyl-5-O-triphenylmethyl-D-erythro-pentofuranoside** was prepared in the manner described in Example 5, with a yield of 87% of theoretical.

**alpha/beta anomer mixture**
**$^{13}$C-NMR (DMSO-$d_6$)**
105.1/104.9 (C-1), 38.7 (C-2), 81.3/80.7 (C-3), 82.6/82.5 (C-4), 63.7/63.2 (C-5); 55.0/54.6 ($OCH_3$); 37.7 (OMs); 86.5/127.6/128.3/128.5/129.6/ 144.1 (OTr)

**Example 10:**

Starting from methyl 5-O-benzyl-2-deoxy-D-erythro-pentofuranoside and using 1.5 equivalents of p-toluene sulfonic acid chloride, **methyl 5-O-benzyl-2-deoxy-3-O-(p-toluenesulfonyl)-D-erythro-pentofuranoside** was prepared in the manner described in Example 5, with a yield of 95% of theoretical.

**alpha/beta-anomer mixture**
**$^{13}$C-NMR ($CDCl_3$)**
105.4/105.7 (C-1), 39.5 (C-2), 80.6/82.1 (C-3), 82.3/82.6 (C-4), 73.6/73.9 (C-5); 55.4/55.6 ($OCH_3$); 21.8/22.0/69.4/70.9/127.7/128.6/128.7/129.2/130.7/1-31.1/138.5/142.2 (OBn and OTs)

**Example 11:**

Starting from methyl 5-O-tert.butyldimethylsilyl-2-deoxy-D-erythro-pentofuranoside, **methyl 5-O-tert.butyldimethylsilyl-2-deoxy-3-O-methanesulfonyl-D-erythro-pentofuranoside** was prepared in the manner described in Example 5, with a yield of 93% of theoretical.

**alpha/beta-anomer mixture**
**$^{13}$C-NMR ($CDCl_3$)**
105.9/105.7 (C-1), 39.9 (C-2), 82.1/80.7 (C-3), 84.5/84.6 (C-4), 63.8/6.31 (C-5); 55.7/55.4 ($OCH_3$); 38.8/38.5 (OMs); 26.1/19.3/-5.4/-5.3 (OTBDMS)

**Example 12:**

A solution of 1.2 ml (10 mmol) of benzoyl chloride in 10 ml dichloromethane was added slowly dropwise with stirring at 5°C to a solution of 1.48 g (10 mmol) of methyl 2-deoxy-D-erythro-pentofuranoside in a mixture of 30 ml absolute dichloromethane and 2.5 ml (31 mmol) absolute pyridine. The cooling bath was then removed and the mixture allowed to stand overnight at room temperature. 1.0 ml of pyridine was then added and the mixture cooled to 5°C, and 1.2 ml (15.6 mmol) of methane sulfonic acid chloride were added dropwise. After standing overnight at room temperature, 1 ml of water was added and, after another hour, the organic phase was extracted with aqueous solutions of $KHSO_4$ and $NaHCO_3$ and with water, dried over sodium sulphate and evaporated. This gave 3.2 g (9.7 mmol), i.e. 97% of theoretical, of **methyl 5-O-benzoyl-2-deoxy-3-O-methanesulfonyl-D-erythro-pentofuranoside,** which was used further without additional purification.

**alpha/beta-anomer mixture**
**$^{13}$C-NMR ($CDCl_3$)**
105.5/105.9 (C-1), 39.9/40.1 (C-2), 79.6/80.8/81.4/82.1 (C-3, C-4), 64.0/64.8 (C-5); 55.7/55.8 ($OCH_3$); 129.4/130.7/134.3/168.0 (OBz); 38.8/39.0 (OMs)

**Example 13:**

Using 4-methylbenzoyl chloride, **methyl 2-deoxy-3-O-methanesulfonyl-5-O-(4-methylbenzoyl)-D-erythro-pentofuranoside** was prepared in the manner described in Example 12, with a yield of 95% of theoretical.

**alpha/beta-anomer mixture**
**$^{13}$C-NMR (DMSO-$d_6$)**
105.2/104.9 (C-1), 38.9 (C-2), 81.1/80.5 (C-3), 81.5/81.1 (C-4), 64.1/63.7 (C-5); 54.8/54.6 ($OCH_3$); 37.5 (OMs);
21.1/129.7/129.8/129.9/130.4/130.9/144.5/166.3 (OMeBz)

**Example 14:**

Using 4-phenylbenzoyl chloride, **methyl 2-deoxy-3-O-methanesulfonyl-5-O-(4-phenylbenzoyl)-D-erythro-pentofuranoside** was prepared in the manner described in Example 12 with a yield of 86% of theoretical.

**alpha/beta-anomer mixture**
**$^{13}$C-NMR ($CDCl_3$)**
105.9/105.5 (C-1), 40.1/39.9 (C-2), 81.5/79.7/82.1/80.9 (C-3, C-4), 64.8/64.0 (C-5); 55.9/55.8 ($OCH_3$); 39.0/38.8 (OMs); 128 - 132/140 - 141/147 - 148/164 (4-PhBzO).

**Example 15:**

Using isobutyloxycarbonyl chloride, **methyl 2-deoxy-5-O-isobutyloxycarbonyl-3-O-**

**methanesulfonyl-D-erythro-pentofuranoside** was prepared in the manner described in Example 12, with a yield of 95% of theoretical.

**alpha/beta-anomer mixture**
**$^{13}$C-NMR ($CDCl_3$)**
105.5/105.9 (C-1), 39.8/40.1 (C-2), 79.5/81.1/81.2/81.5 (C-3, C-4), 66.6/67.8 (C-5); 55.7/55.8 ($OCH_3$); 19.2/28.1/74.5/75.9/158.0 (OIBOC); 38.6/38.9 (OMs)

**Example 16:**

0.1 g (0.4 mmol) of pyridiniumtoluene-4-sulfonate was added with stirring to a solution of 1.48 g (10 mmol) of methyl 2-deoxy-D-erythro-pentofuranosideand 0.92 g, (11 mmol) of 3.4-dihydro-2H-pyran in 50 ml of dichloromethane. After stirring overnight at room temperature, the reaction solution was cooled to 0° C and mixed with 2.2 g (22 mmol) of triethylamine and 1.7 g (15 mmol) of methanesulfonic acid chloride. After warming to room temperature, the mixture was stirred for 10 hours and then mixed with 1 ml of water. After stirring for another hour, the organic phase was extracted with aqueous solutions of $KHSO_4$ and $NaHCO_3$, dried over sodium sulphate and evaporated in a rotary evaporator. This gave 2.9 g (9.3 mmol), i.e. 93% of theoretical, of **methyl 2-deoxy-3-O-methanesulfonyl-5-O-tetrahydropyranyl-D-erythro-pentofuranoside.**

**alpha-anomer**
**$^{13}$C-NMR ($CDCl_3$)**
105.6/105.6 (C-1, 39.9/40.0 (C-2), 80.6 (C-3), 82.7/82.8 (C-4), 66.8/66.9 (C-5); 55.6 ($OCH_3$); 19.6/19.8/25.6/25.7/30.7/30.8/62.7/63.0/99.5/100.0 (OTHP); 38.9 (OMs)

**beta-anomer**
**$^{13}$C-NMR ($CDCl_3$)**
105.8/106.1 (C-1), 39.5/40.2 (C-2), 80.6/81.8/82.5/82.8 (C-3, C-4), 66.5/67.2 (C-5); 55.7 ($OCH_3$);
19.6/19.8/25.7/25.9/30.8/31.1/62.5/63.0/99.8(b) (OTHP); 39.0 (OMs)

**Example 17:**

A solution of 0.3 g (1 mmol) of methyl 2-deoxy-2,3-di-O-methanesulfonyl-D-erythro-pentofuranoside, prepared according to Example 33, in 6 ml of N,N-dimethylformamide was mixed with 0.3 g (2 mmol) of sodium benzoate and kept at 80° C for 10 hours with stirring. After distilling off the solvent, the residue was taken up in 20 ml of dichloromethane and 10 ml of water; the organic phase was dried over sodium sulphate and evaporated in a rotary evaporator. This gave 0.3 g (0.9 mmol), i.e. 90% of theoretical, of **methyl 5-O-benzoyl-2-deoxy-3-O-methanesulfonyl-D-erythro-pentofuranoside**, the physical data of which agreed with that of Example 12.

**Example 18:**

3.10 g (10 mmol) of methyl 2-deoxy-3-O-methanesulfonyl-5-O-pivaloyl-D-erythro-pentofuranoside, prepared according to Example 5, were dissolved in 40 ml of N,N-dimethylformamide, mixed with 2.9 g (20 mmol) of sodium benzoate and heated to about 90° C. After a reaction time of 8 hours, the solvent was removed in a rotary evaporator, the residue was taken up in 100 ml of dichloromethane, the organic phase was extracted twice with aliquots of 15 ml water, dried over sodium sulphate, filtered and evaporated in a rotary evaporator. This gave 2.8 g (8.3 mmol), i.e. 83% of theoretical, of **methyl 3-O-benzoyl-2-deoxy-5-O-pivaloyl-D-threo-pentofuranoside.**

**alpha-anomer**
**$^{1}$H-NMR ($CDCl_3$)**
5.25 (H-1, J 2.5, 5.5), 2.44 (H-$2_a$, J 2.5, 6.5, 14.0), 2.33 (H-$2_b$, J 3.5, 5.5, 14.0), 5.68 (H-3, J 3.5, 3.5, 6.5), 4.2-4.4 (H-4, H-$5_a$, H-$5_b$, m);
1.15, 1.18, 1.21 (each $CH_3$), 3.39 ($OCH_3$), 7.4-8.2 (Ph)
**$^{13}$C-NMR ($CDCl_3$)**
104.7 (C-1), 40.9 (C-2), 74.4 (C-3), 77.0 (C-4), 62.3 (C-5); 55.5 ($OCH_3$); 27.2/38.9/179.5/129.2/130.3/134.1/166.6 (OBz and OPiv)

**beta-anomer**
**$^{1}$H-NMR ($CDCl_3$)**
5.12 (H-1, J 4.2), 2.25 (H-$2_a$, J 13.5), 2.47 (H-2b, J 4.2, 5.5, 13.5), 5.69 (H-3, J 5.5, 5.5), 4.52 (H-4, J 5.5, 5.5, 5.5), 4.39 (H-$5_a$, J 5.5), 4.37 (H-$5_b$, J 5.5); 1.12, 1.20, 1.25 (each $CH_3$), 3.41 ($OCH_3$), 7.4-8.1 (Ph)
**$^{13}$H-NMR ($CDCl_3$)**
105.7 (C-1), 40.2 (C-2), 73.2 (C-3), 79.4 (C-4), 64.3 (C-5), 55.7 ($OCH_3$); 27.4/39.0/179.0/129.4/130.6/131.2/134.3/134.7/167.0 (OBz and OPiv)

**Example 19:**

Starting from methyl 2-deoxy-3-O-methanesulfonyl-5-O-triphenylmethyl D-erythro-pentofuranoside and using 2 equivalents of potassium acetate, **methyl 3-O-acetyl-2-deoxy-5-O-triphenylmethyl D-threo-pentofuranoside** was prepared in the manner described in Example 18, with a yield of 84% of theoretical.

**alpha-anomer**
**$^{13}C$-NMR (DMSO-$d_6$)**
104.0 (C-1), 39.5 (C-2), 73.2 (C-3), 77.6 (C-4), 61.2 (C-5); 54.9 $OCH_3$); 20.4/171.0 (OAc); 87.5/127.6/128.4/128.5/128.7/144.1 (OTr)

**beta-anomer**
**$^{13}C$-NMR (DMSO-$d_6$)**
104.5 (C-1), 38.6 (C-2), 72.1 (C-3), 79.8 (C-4), 63.0 (C-5); 54.8 ($OCH_3$); 20.8/170.5 (OAc); 86.5/127.6/128.3/128.4/128.7/144.2 (OTr)

**Example 20:**

Starting from methyl 5-O-benzyl-2-deoxy-3-O-(p-toluenesulfonyl)-D-erythro-pentofuranoside and using 2 equivalents of potassium acetate, **methyl 3-O-acetyl-5-O-benzyl-2-deoxy-D-threo-pentofuranoside** was prepared in the manner described in Example 18, with a yield of 87% of theoretical.

**alpha/beta-anomer mixture**
**$^{13}C$-NMR ($CDCl_3$)**
105.5/105.8 (C-1), 39.7/39.7 (C-2), 73.8/74.1/80.6/82.1 (C-3, C-4), 70.7/71.0 (C-5); 55.5/55.7 ($OCH_3$); 20.4/20.7/170.4/170.8/(OAc)-;69.5/71.0/128.5/128.7/129.4/130.8/139.0 (OBn)

**Example 21:**

1.68 g (5 mmol) of methyl 3-O-benzoyl-2-deoxy-5-O-pivaloyl-D-threo-pentofuranoside, prepared according to Example 18, were dissolved in 20 ml of $NH_3$-saturated methanol and heated to 50 °C. After about 4 hours the solvent was evaporated and the residue purified by chromatography (ethyl acetate/toluene = 3/1). This gave 0.86 g (3.7 mmol), i.e. 74% of theoretical, of **methyl 2-deoxy-5-O-pivaloyl-D-threo-pentofuranoside.**

**alpha-anomer**
**$^{13}H$-NMR ($CDCl_3$)**
5.17 (H-1, J 3.5, 5.3), 2.22 (H-$2_a$, J 2.2, 5.3, 14.0), 2.14 (H-$2_b$, J 3.5, 5.8, 14.0), 4.30(H-3, J 2.2, 3.5, 5.8), 4.03 (H-4, J 3.5, 5.1, 7.0), 4.53 (H-$5_a$, J 7.0, 11.5), 4.19 (H-$5_b$, J 5.1, 11.5); 3.36 ($OCH_3$), 1.21 (3 $CH_3$)
**$^{13}C$-NMR ($CDCl_3$)**
105.0 (C-1), 42.8 (C-2), 71.7 (C-3), 79.3 (C-4), 62.4 (C-5); 55.9 ($OCH_3$); 27.4/39.5/180.5 (OPiv)

**beta-anomer**
**$^1H$-NMR ($CDCl_3$)**
5.08(H-1, J 4.2), 2.17 (H-$2_a$, J 13.7), 2.13 (H-$2_b$, J 4.2, 8.7, 13.7), 4.30 (H-3, n.r.), 4.16 (H-4 J 4.1, 5.3, 6.9), 4.42 (H-$5_a$, J 5.3, 11.6), 4.29 (H-$5_b$, J 6.9, 11.6); 3.37 ($OCH_3$), 1.21 ($3CH_3$)
**$^{13}C$-NMR ($CDCl_3$)**
105.8 (C-1), 41.7 (C-2), 71.9 (C-3), 82.6 (C-4), 64.6 (C-5); 55.4 ($OCH_3$); 27.4/39.5/180.0 (OPiv)

**Example 22:**

A solution of 2.16 g (5 mmol) methyl 3-O-acetyl-2-deoxy-5-O-triphenylmethyl D-threo-pentofuranoside, prepared according to Example 19, in 20 ml of absolute methanol was mixed with 1 ml of 1-molar sodium methylate solution in absolute methanol. After one hour, it was neutralized with an ion exchanger (Amberlite IR 120 ($H^+$)), the solvent was evaporated and the residue purified by chromatography (petroleum ether/ethyl acetate = 5/1). This gave 1.6 g (4.1 mmol), i.e. 82% of theoretical, of **methyl 2-deoxy-5-O-triphenylmethyl D-threo-pentofuranoside.**

**alpha-anomer**
**$^1H$-NMR ($CDCl_3$)**
5.17 (H-1, J 4.3, 4.3), 2.16 (H-$2_a$, J 4.3, 4.3), 2.16 (H-$2_b$, J 4.3, 4.3), 4.54 (H-3, J 4.3, 4.3, 4.4), 4.18 (H-4, J 4.4, 4.4, 6.8), 3.47 (H-$5_a$, J 4.4, 9.7), 3.34 (H-$5_b$, J 6.8, 9.7); 3.35 ($OCH_3$), 7.2 - 7.5 (3Ph)
**$^{13}C$-NMR ($CDCl_3$)**
105.3 (C-1), 42.7 (C-2), 72.8 (C-3), 79.4 (C-4), 62.7 (C-5); 55.8 ($OCH_3$), 87.9/128.1/128.8/129.2/144.5 (OTr)

**beta-anomer**
**$^1H$-NMR ($CDCl_3$)**
5.09 (H-1, J 3.0), 2.08 (H-$2_a$, H-2b, bs), 4.15 - 4.25 (H-3, H-4, m), 3.4 - 3.45 (H-$5_a$, H-$5_b$, m); 3.33 ($OCH_3$), 7.2 - 7.5 (3Ph)
**$^{13}C$-NMR ($CDCl_3$)**
105.9 (C-1), 41.8 (C-2), 72.3 (C-3), 84.6 (C-4), 64.8 (C-5); 55.5 ($OCH_3$), 87.4/128.0/128.7/129.7/145.0 (OTr)

**Example 23:**

Starting from methyl 3-O-acetyl-5-O-benzyl-2-deoxy-D-threo-pentofuranoside, **methyl 5-O-benzyl-2-deoxy-D-threo-pentofuranoside** was prepared in the manner described in Example 22, with a yield of 97% of theoretical.

**alpha/beta-anomer mixture**
**$^{13}C$-NMR ($CDCl_3$)**
105.0/105.6 (C-1), 41.7/43.0 (C-2), 73.9/74.0 (C-3), 79.4/83.8 (C-4), 71.9/72.2 (C-5); 55.5/55.7 ($OCH_3$); 69.4/70.7/128.4/128.5/129.1/139.0 (OBn)

**Example 24:**

15.6 g (0.23 mole) of $NaNO_2$ were mixed into 160 ml of 1,3-dimethyl 3,4,5,6-tetrahydro-2(1H)-pyrimidinone (DMPU) and the mixture heated to 130°C. 46.5 g (0.15 mole) of methyl 2-deoxy-3-O-methanesulfonyl-5-O-pivaloyl-D-erythro-pentofuranoside dissolved in 30 ml of DMPU were added slowly dropwise to this reaction mixture with vigorous stirring. After 3 hours, the reaction mixture was mixed with 30 ml of dichloromethane and 400 ml of iced water, the organic phase was extracted with water, dried and evaporated in a rotary evaporator. The residue was purified by column chromatography (cyclohexane/ethyl acetate = 2/1). This gave 25.0 g (0.11 mole), i.e. 72% of theoretical, of **methyl 2-deoxy-5-O-pivaloyl-D-threo-pentofuranoside**, the physical data of which agreed with those of Example 21.

**Example 25:**

Starting from methyl 5-O-benzoyl-2-deoxy-3-O-methanesulfonyl-D-erythro-pentofuranoside, **methyl 5-O-benzoyl-2-deoxy-D-threo-pentofuranoside** was obtained in the same manner as in Example 24, with a yield of 74%.

**alpha/beta-anomer mixture**
**$^{13}$C-NMR ($CDCl_3$)**
105.2/105.9 (C-1), 41.9/42.8 (C-2), 71.8/72.2 (C-3), 79.3/82.8 (C-4), 65.0/65.4 (C-5); 55.6/56.0 ($OCH_3$); 129.3/130.5/130.7/134.1/168.0 (OBz)

**Example 26:**

Starting from methyl 2-deoxy-3-O-methanesulfonyl-5-O-(4-methylbenzoyl)-D-erythro-pentofuranoside, **methyl 2-deoxy-5-O-(4-methylbenzoyl)-D-threo-pentofuranoside** was obtained in the same manner as described in Example 24, with a yield of 78%.

**alpha/beta-anomer mixture**
**$^{13}$C-NMR (DMSO-$d_6$)**
104.8/105.5 (C-1), 42.2/41.0 (C-2), 70.1/69.6 (C-3), 79.8/78.6 (C-4), 65.3/64.0 (C-5); 54.7/54.5 ($OCH_3$); 21.1/129.6/129.7/129.8/130.4/130.9/144.0/166.5 (OMeBz)

**Example 27:**

Starting from methyl 2-deoxy-5-O-isobutyloxycarbonyl-3-O-methane-sulfonyl-D-erythro-pentofuranoside, **methyl 2-deoxy-5-O-isobutyloxycarbonyl-D-threo-pentofuranoside** was obtained in the same manner as described in Example 24, with a yield of 78%.

**alpha/beta-anomer mixture**
**$^{13}$C-NMR ($CDCl_3$)**
105.1/105.7 (C-1), 41.6/42.7 (C-2), 71.3/71.6 (C-3), 78.9/82.1 (C-4), 67.2/68.2(C-5); 55.4/55.6 ($OCH_3$); 19.0/27.9/74.5/74.7/157.8 (OIBOC)

**Example 28:**

Starting from methyl 2-deoxy-3-O-methanesulfonyl-5-O-tetrahydropyranyl-D-erythro-pentofuranoside, **methyl 2-deoxy-5-O-tetrahydropyranyl-D-threo-pentofuranoside** was obtained in the same manner as in Example 24, with a yield of 72%.

**alpha/beta-anomer mixture**
**$^{13}$C-NMR ($CDCl_3$)**
too complex;
19.5/25.5/31.0/63.0/99.5 (all b) (OTPH)

**Example 29:**

Starting from methyl 5-O-tert.butyldimethylsilyl-2-deoxy-3-O-methanesulfonyl-D-erythro-pentofuranoside, **methyl 5-O-tert.butyldimethylsilyl-2-deoxy-D-threo-pentofuranoside** was obtained in the same manner as in Example 24, with a yield of 79%.

**alpha-anomer**
**$^{13}$C-NMR (DMSO-$d_6$)**
104.2 (C-1), 42.5 (C-2), 69.9 (C-3), 82.5 (C-4), 62.2 (C-5); 54.5 ($OCH_3$); 0.1/19.0/25.7 (OTBDMS)

**beta-anomer**
**$^{13}$C-NMR (DMSO-$d_6$)**
104.5 (C-1), 41.1 (C-2), 69.3 (C-3), 83.6 (C-4), 63.4 (C-5); 54.4 ($OCH_3$); 0.1/19.0/25.8 (OTBDMS)

**Example 30:**

A solution of 1.16 g (5 mmol) of methyl 2-deoxy-5-O-pivaloyl-D-erythro-pentofuranoside in 15 ml of absolute dichloromethane and 1.0 ml (12 mmol) pyridine was mixed slowly with stirring and cooling in ice with a solution of 0.9 ml (5.3 mmol) of trifluoromethane sulfonic acid anhydride in 5 ml of absolute dichloromethane. After 15 minutes, the organic phase was extracted with aqueous solutions of $KHSO_4$ and $NaHCO_3$ and then with iced water, dried over sodium sulphate and evaporated in a vacuum to about one-quarter of the original volume, mixed with 10 ml of N,N-dimethylformamide and 1.0 g (14.5 mmol) sodium nitrite and stirred for 2 hours at 40°C. The solvent was then evaporated in a rotary evaporator, the residue was mixed with 25 ml of dichloromethane and 15 ml of water, the organic phase was dried over sodium sulphate, evaporated and the residue purified by chromatography (ethyl acetate/toluene = 3/1). This gave 0.84 g (3.6 mmol), i.e. 72% of theoretical, of

**methyl 2-deoxy-5-O-pivaloyl-D-threo-pentofuranoside**, the physical data of which agreed with those of Example 21.

**Example 31:**

Starting from methyl 2-deoxy-5-O-triphenylmethyl D-erythro-pentofuranoside, **methyl 2-deoxy-5-O-triphenylmethyl D-threo-pentofuranoside** was obtained in the same manner as described in Example 30, with a yield of 82%; the physical data agreed with those of Example 22.

**Example 32:**

A solution of 0.3 g (1 mmol) of methyl 2-deoxy-3,5-di-O-methanesulfonyl-D-erythro-pentofuranoside, prepared according to Example 33, dissolved in 6 ml of N,N-dimethylformamide was mixed with 0.17 g (1.2 mmol) of sodium benzoate and stirred for 24 hours at 80° C. After adding 0.7 g (10 mmol) of sodium nitrite, the temperature was increased to 120° C and stirred for a further 3 hours. After distilling off the solvent, the residue was taken up in 30 ml of dichloromethane and 30 ml of water, and the organic phase was extracted with water, dried over sodium sulphate, evaporated and purified by chromatography (petroleum ether/ethyl acetate = 1/1). This gave 0.18 g (0.72 mmol), i.e. 72% of theoretical, of **methyl 5-O-benzoyl-2-deoxy-D-threo-pentofuranoside**, the physical data of which agreed with those of Example 25.

**Example 33:**

A solution of 2.5 ml (32.0 mmol) of methane sulfonic acid chloride in 10 ml of absolute dichloromethane was added dropwise to a solution of 1.48 g (10 mmol) of methyl 2-deoxy-D-erythro-pentofuranoside in 20 ml of absolute dichloromethane and 5 ml (61.8 mmol) of pyridine, whereby the reaction mixture heated up. It was then heated under reflux for about 2 hours. After cooling to room temperature, 2 ml of water were added and the mixture stirred for one hour at room temperature. The organic phase was then extracted with an aqueous solution of $KHSO_4$ and $NaHCO_3$ and with water, dried over sodium sulphate and evaporated in a rotary evaporator. This gave 2.9 g (9.5 mmol), i.e. 95% of theoretical, of **methyl 2-deoxy-3,5-di-O-methanesulfonyl-D-erythro-pentofuranoside.**

**alpha/beta-anomer mixture**
**$^{13}$C-NMR ($CDCl_3$)**
105.5/106.0 (C-1), 39.6/39.8 (C-2), 78.9/80.2 (C-3), 81.0/81.4 (C-4), 68.6/69.0 (C-5); 55.7/55.9 ($OCH_3$); 37.9/38.6 (OMs)

**Example 34:**

5.9 g (60 mmol) of potassium acetate were added to a solution of 3.04 g (10 mmol) of methyl 2-deoxy-3,5-di-O-methanesulfonyl-D-erythro-pentofuranoside, prepared according to Example 33, in 50 ml of N,N-dimethylformamide, and the mixture was stirred at 90° C overnight. After distilling off the solvent under vacuum, 50 ml of water and 100 ml of dichloromethane were added, the organic phase was washed once with 20 ml of water, dried over sodium sulphate and evaporated in a rotary evaporator. The residue was purified by column chromatography (toluene/ethyl acetate = 2/1). This gave 1.95 g (8.4 mmol), i.e. 84% of theoretical, of **methyl 3,5-di-O-acetyl-2-deoxy-D-threo-pentofuranoside.**

**alpha-anomer**
**$^{1}$H-NMR ($CDCl_3$)**
5.17 (H-1, J 2.6, 5.6), 2.30 (H-$2_a$, J 2.6, 6.6, 14.7), 2.18 (H-$2_b$, J 3.0, 5.6, 14.7), 5.43 (H-3, J 3.0, 4.1, 6.6), 4.18 - 4.32 (H-4, H-$5_a$, m), 4.08 (H-$5_b$,J 5.7, 11.6, 17.3); 3.38 ($OCH_3$); 2.10 (2 $CH_3$)
**$^{13}$C-NMR ($CDCl_3$)**
104.8 (C-1), 40.8 (C-2), 74.0 (C-3), 76.9 (C-4), 62.7 (C-5); 55.8 ($OCH_3$); 21.1/171.3/171.8 (OAc)

**beta-anomer**
**$^{1}$H-NMR ($CDCl_3$)**
5.07 (H-1, J 1.1, 5.5), 2.40 (H-$2_a$, J 5.5, 7.2, 14.7), 2.10 (H-$2_b$, J 1.1, 2.1, 14.7), 5.41 (H-3, J 2.1, 3.6, 7.2), 4.2 - 4.45 (H-4, H-$5_a$, H-$5_b$, m); 3.39 ($OCH_3$); 2.10 (2 $CH_3$).
**$^{13}$C-NMR ($CDCl_3$)**
105.4 (C-1), 39.9 (C-2), 72.7 (C-3) 79.1 (C-4), 64.3 (C-5); 55.8 ($OCH_3$); 21.2/21.3/171.7/171.9 (OAc)

**Example 35:**

1.16 g (5 mmol) of methyl 3,5-di-O-acetyl-2-deoxy-D-threo-pentofuranoside in 20 ml of absolute methanol were mixed with 1 ml of a 1 molar sodium methylate solution in absolute methanol, and stirred for about one hour. After this, the solution was neutralized with an ion exchanger (Amberlite IR 120 ($H^+$)), the solvent was evaporated off and the residue chromatographed (petroleum ether/ethyl acetate = 5/1). This gave 0.67 g (4.5 mmol),. i.e. 90% of theoretical, of **methyl 2-deoxy-D-threo-pentofuranoside.**

**alpha/beta-anomer mixture**
**$^{13}$C-NMR (DMSO-$d_6$)**
104.5/104.3 (C-1), 42.4/41.1 (C-2), 70.1/69.4 (C-3), 83.6/82.1 (C-4), 61.0/59.9 (C-5); 54.6/54.5 ($OCH_3$)

**Example 36:**

1.52 g (5 mmol) of methyl 2-deoxy-3,5-di-O-methanesulfonyl-D-erythro-pentofuranoside, prepared according to Example 33, were stirred with 1.38 g (20 mmol) of sodium nitrite in 15 ml of N,N-dimethylformamide for 13 hours at 120 °C. After cooling to room temperature and distilling off the solvent under vacuum, the solid residue was digested twice with aliquots of 30 ml acetone, filtered and the solvent evaporated. This gave 0.70 g (4.75 mmol), i.e. 95% of theoretical, of **methyl 2-deoxy-D-threo-pentofuranoside**, the physical data of which agreed with those of Example 35.

**Example 37:**

A solution of 3.90 g (10 mmol) of methyl 2-deoxy-5-O-triphenylmethyl D-threo-pentofuranoside, prepared according to Example 22, in 40 ml of absolute dichloromethane was mixed with 1.25 ml (10 mmol) of bortrifluoride-diethylether complex and 2.5 ml (98.6 mmol) of absolute methanol, and stirred for half an hour at room temperature, after which about half the solvent was distilled off under vacuum. By mixing with petroleum ether, 1.3 g (8.8 mmol), i.e. 88% of theoretical, of **methyl 2-deoxy-D-threo-pentofuranoside** was precipitated, the physical data of which agreed with those of Example 35.

**Example 38:**

A solution of 1.26 g (5 mmol) of methyl 2-deoxy-5-O-benzoyl-D-threo-pentofuranoside in 15 ml of absolute methanol was mixed at room temperature with 0.5 ml (0.5 mmol) of a 1 molar sodium methanolate solution in absolute methanol. After one hour, the solution was neutralized with the aid of the ion-exchanger Amberlite IR 120 ($H^+$) and the solvent evaporated. This gave 0.70 g (4.7 mmol), i.e. 94% of theoretical, of **methyl 2-deoxy-D-threo-pentofuranoside**, the physical of which agreed with those of Example 35.

**Example 39:**

1.48 g (10 mmol) of methyl 2-deoxy-D-threo-pentofuranoside were dissolved in a mixture of 10 ml dioxan and 20 ml water, mixed with 0.5 ml of trifluoroacetic acid and kept at 35 °C with stirring. After about 6 hours, it was neutralized with the ion-exchanger Merck III ($OH^-$) and the solution evaporated in a rotary evaporator. This gave 1.18 g (8.8 mmol), i.e. 88% of theoretical, of **2-deoxy-D-threo-pentose.**

**$^{13}$C-NMR ($D_2O$)**

97.6/95.2 (C-1), 42.1/39.7 (C-2), 73.7/73.6/73.2/71.4/68.3/65.9 (C-3, 4, 5)

**Example 40:**

4.64 g (20 mmol) of methyl 2-deoxy-5-O-pivaloyl-alpha-D-threo-pentofuranoside, prepared according to Example 24, were converted in the manner described in Example 5 to 5.83 g (18.8 mmol) of **methyl 2-deoxy-3-O-methanesulfonyl-5-O-pivaloyl-alpha-D-threo-pentofuranoside**, i.e. 94% of theoretical.

**$^{13}$C-NMR ($CDCl_3$)**

104.6 (C-1), 41.4 (C-2), 77.2 (C-3), 79.8 (C-4), 61.7 (C-5); 55.9 ($OCH_3$); 27.4/40.2/179.5 (OPiv); 38.9 (OMs)

**Example 41:**

9.0 g (29 mmol) of methyl 2-deoxy-3-O-methanesulfonyl-5-O-pivaloyl-alpha-D-threo-pentofuranoside, prepared according to Example 40, were dissolved in 70 ml of N,N-dimethylformamide, then 7.8 g (120 mmol) of sodium aside were added and the mixture heated to 110 °C for 3 hours with stirring. After evaporating the solvent under vacuum, the residue was distributed between dichloromethane and water, the organic phase was dried over sodium sulphate and evaporated to a syrup. This gave 6.8 g, i.e. 91% of theoretical of **methyl 3-azido-2,3-dideoxy-5-O-pivaloyl-alpha-D-erythro-pentofuranoside.**

**$^{1}$H-NMR ($CDCl_3$)**

5.08 (H-1. J 1.2, 5.2), 2.40 (H-$2_a$, J 5.2, 8.9, 14.2), 2.06 (H-$2_b$, J 1.2, 3.3, 14.2), 3.87 (H-3, J 3.3, 4.9, 8.9), 4.1 - 4.3 (H-4, H-5a, H-$5_b$, m); 3.38 ($OCH_3$), 1.21(3 $CH_3$)

**$^{13}$C-NMR ($CDCl_3$)**

104.8 (C-1), 38.6 (C-2), 61.0 (C-3), 80.4 (C-4), 63.6 (C-5), 55.0 ($OCH_3$); 27.0/38.8/178.7 (OPiv)

**Example 42:**

A solution of 3.4 g (13.2 mmol) of methyl 3-azido-2,3-dideoxy-5-O-pivaloyl-alpha-D-erythro-pentofuranoside, prepared according to Example 41, in 20 ml of absolute methanol was mixed at room temperature with 13 ml of 1 N sodium methanolate solution and allowed to stand overnight. After neutralization with the ion-exchanger IR 120 ($H^+$) and evaporating the solvent, 2.0 g, i.e. 87% of theoretical, of **methyl 3-azido-2,3-dideoxy-alpha-D-erythro-pentofuranoside** were obtained.

**$^{1}$H-NMR ($CDCl_3$)**

5.09 (H-1, J 1.5, 5.3), 2.40 (H-2a, J 5.3, 8.6, 14.2), 2.03 (H-$2_b$, J 1.5, 3.4, 14.2), 3.95 - 4.05 (H-3, H-4, m), 3.82 (H-$5_a$, J 3.3, 12.0), 3.69 (H-$5_b$, 3.5, 12.0); 3.39 ($OCH_3$)

**$^{13}$C-NMR ($CDCl_3$)**
104.9 (C-1), 38.8 (C-2), 59.9 (C-3), 82.8 (C-4), 62.1 (C-5), 55.0 ($OCH_3$)

**Example 43:**

A solution of 1.73 g (10 mmol) of methyl 3-azido-2,3-dideoxy-alpha-D-erythro-pentofuranoside in a mixture of 10 ml dioxan and 10 ml water was mixed with 3 ml of trifluoroacetic acid and allowed to stand overnight at room temperature. After neutralization with the ion-exchanger Merck III ($OH^-$) and removal of the solvent, 1.46 g, i.e. 92% of theoretical, of **3-azido-2,3,dideoxy-D-erythro-pentose** was obtained.
**$^{13}$C-NMR ($D_2O$)**
96.9/94.4 (C-1), 34.8/33.9 (C-2), 61.3/59.4 (C-3), 69.8/68.6 (C-4), 66.3/64.7 (C-5)

**Example 44:**

8.73 g (33.9 mmol) of methyl 3-azido-2,3-dideoxy-5-O-pivaloyl-alpha-D-erythro-pentofuranoside were dissolved in 50 ml of acetonitrile. 11.9 g (44.0 mmol) of 5-methyl 2,4-bis-(trimethylsilyloxy)pyrimidine and 7.5 ml (39.0 mmol) of trimethylsilyltrifluoromethane sulfonate were added to this solution at 0°C with stirring. After about 30 minutes the mixture was warmed to room temperature and, after a further 2 hours, was stirred into 300 ml of iced water. The mixture was extracted three times with aliquots of 200 ml dichloromethane and the excess precipitated thymine was filtered off. The cooled organic phases were washed with 100 ml water, dried over sodium sulphate and evaporated in a rotary evaporator. The residue was dissolved in 100 ml of absolute methanol and mixed with 11.4 ml of 30% sodium methylate solution. After stirring for one hour at room temperature, the solution was mixed with 53 ml of highly acidic ion-exchanger (IR 120, $H^+$ form) and stirred until a neutral reaction was obtained. The solution was filtered off from the ion exchanger and evaporated to dryness. The residue was purified by column chromatography on silica gel (ethyl acetate) and crystallized from cyclohexane/isopropyl alcohol. This gave 2.98 g (11 mmol), i.e. 33% of theoretical, of **3'-azido-3'-deoxy-thymidine.**
**$^{1}$H-NMR (DMSO-$d_6$)**
7.69 (H-6), 1.80 ($CH_3$), 6.10 (H-1', J 6.4, 6.4), 2.2 - 2.45 (H-$2_a$, H-$2_b$, m), 4.41 (H-4', J 5.1, 5.1, 7.1), 3.82 (H-3', 3.8, 3.8, 5.1), 3.63 (H-$5_a$, H-$5_b$, bs), 5.24 and 3.37 (NH and OH)
**$^{13}$C-NMR (DMSO-$d_6$)**
164.4 (C-4), 151.0 (C-2), 136.6 (C-6), 109.9 (C-5), 12.1 ($CH_3$); 84.2 and 83.6 (C-1' and C-4'), 60.9 and 60.3 (C-5' and C-3'), 36.2 (C-2')

**Example 45:**

32.9 g (0.14 mole) methyl 2-deoxy-5-O-pivaloyl-D-threo-pentofuranoside were dissolved in 230 ml dichloromethane, and the mixture was cooled to -5°C and 25 g pyridine added. 32.3 g (0.154 mole) trifluoromethanesulfonic acid anhydride dissolved in 700 ml dichloromethane were slowly added to this solution dropwise with stirring. After stirring further for about 1 h ice water was added to the solution and the excess pyridine was removed by acidification with 10% hydrochloric acid and washing several times with water. The organic phase was dried over sodium sulphate, filtered free of drying agent and cooled to -25°C. 168 ml 1 M solution of tetrabutylammonium fluoride in tetrahydrofuran was added dropwise with stirring over a period of 1 h. After 24 h of further stirring at room temperature the solvent was evaporated on the rotary evaporator and the residue extracted several times with ether. The combined organic phases were washed with water, dried over sodium sulphate and evaporated on the rotary evaporator. The residue was purified by column chromatography (petroleum ether:ethyl acetate = 10:1). 17.9 g (75.6 mmol) **methyl 2,3-dideoxy-3-fluoro-5-O-pivaloyl-D-erythro-pentofuranoside** was obtained, i.e. 54% of the theoretical yield.

**alpha-anomer**
**$^{13}$C-NMR ($CDCl_3$)**
105.9 (C-1), 40.1 (C-2, J 20.8), 94.4 (C-3, J 181.1), 82.3 (C-4, J 27.1), 63.9 (C-5, J 9.2); 55.7 ($OCH_3$); 27.5/39.5/179.2 (OPiv)

**beta-anomer**
**$^{13}$C-NMR ($CDCl_3$)**
107.0 (C-1, J 2.9), 40.3 (C-2, J 21.6), 95.5 (C-3, J 180.7), 83.0 (C-4, J 25.0), 64.6 (C-5, J 9.3); 56.2 (OCH3); 27.2/39.5/179.2 (OPiv)

**Example 46:**

Methyl-2-deoxy-5-O-triphenylmethyl D-threo-pentofuranoside was used as starting material to obtain **methyl 2,3-dideoxy-3-fluoro-5-O-triphenylmethyl D-erythro-pentofuranoside** in a 76% yield, by the method described in Example 45.

**alpha-anomer:**
**$^{13}$C-NMR ($CDCl_3$)**
106.8 (C-1), 40.4 (C-2, J 20.6), 95.7 (C-3, J 179.3), 84.7 (C-4, J 25.6), 64.4 (C-5, J 9.9); 56.1 ($OCH_3$); 87.5/128.8/129.5/130.3/145.5 (OTr)

**beta-anomer:**
**$^{13}$C-NMR ($CDCl_3$)**

106.4 (C-1), 39.8 (C-2, J 21.2), 95.4 (C-3, J 177.5), 84.0 (C-4, J 23.6), 64.3 (C-5, J 10.0); 56.0 ($OCH_3$); 87.5/128.0/128.5/129.4/144.7 (OTr)

**Example 47:**

4.64 g (20 mmol) methyl 2-deoxy-5-O-pivaloyl-alpha-D-threo-pentofuranoside prepared according to Example 24, were converted by the method described in Example 5 into 5.83 g (18.8 mmol) **methyl 2-deoxy-3-O-methanesulfonyl-5-O-pivaloyl-alpha-D-threo-pentofuranoside**, i.e. with a yield 94% of the theoretical.

**$^{13}$C-NMR ($CDCl_3$)**

104.6 (C-1), 41.4 (C-2), 77.2 (C-3), 79.8 (C-4), 61.7 (C-5); 55.9 ($OCH_3$); 27.4/40.2/179.5 (OPiv); 38.9 (OMs)

**Example 48:**

A mixture of 3.1 g (10 mmol) methyl 2-deoxy-3-O-methanesulfonyl-5-O-pivaloyl-alpha-D-threo-pentofuranoside, 5.8 g (100 mmol) potassium fluoride and 20 g acetamide was heated for 1 hour at 150 °C with stirring, then cooled to room temperature. The reaction mixture was added dropwise with stirring to 100 ml aqueous sodium hydrogen carbonate solution and this solution was extracted with two lots of 100 ml dichloromethane. The organic phase was dried over sodium sulphate, evaporated and purified chromatographically (ethyl acetate:petroleum ether = 1:5). 0.75 g (3.2 mmol) **methyl 2,3-dideoxy-3-fluoro-5-O-pivaloyl-alpha-D-erythro-pentofuranoside** was obtained, i.e. a yield 32% of the theoretical, whose physical data corresponded to those in Example 45.

**Example 49:**

The splitting off of the protective group $R_7$ from a compound with formula 10 took place, depending on its functional type, either by acid catalysis as described in Example 37 for triphenylmethyl and tetrahydropyranyl groups or by base catalysis as described in Example 38 for acyl, alkyloxycarbonyl and aryloxycarbonyl groups, hydrogenolysis for benzyl ethers or by fluoride ions for silylether groups, in each case an 85-95% yield of **methyl 2,3-dideoxy-3-fluoro-alpha-D-erythro-pentofuranoside** being obtained.

**$^{13}$C-NMR ($D_2O$)**

106.3 (C-1), 39.2 (C-2, J 19.8), 95.1 (C-3, J 174.2), 84.7 (C-4, J 24.7), 61.5 (C-5, J 10.8); 55.3 ($OCH_3$)

**Example 50:**

Methyl-2,3-dideoxy-3-fluoro-D-erythro-pentofuranoside was hydrolysed by the method described in Example 39 to give **2,3-dideoxy-3-fluoro-D-erythropentose**, which was isolated as a mixture of predominantly pyranoid forms with a yield of 91% of the theoretical.

**$^{13}$C-NMR ($D_2O$)**

95.3 (C-1, m), 36.7/36.4 (C-2, J 19.2/19.0), 93.4/92.0 (C-3 J 177.0/172.5), 68.9/68.3 (C-4, J 11.2/14.4), 66.0/64.4 (C-5, J 4.9/8.3)

**Example 51:**

2.34 g (10 mmole) Methyl 2,3-dideoxy-3-fluoro-5-O-pivaloyl-alpha-D-erythro-pentofuranoside, obtained according to Example 44, were dissolved in a mixture of 25 ml acetic acid and 5 ml acetic anhydride and, under cooling and agitation, 0.2 ml concentrated sulphuric acid were added slowly. Alter quantitative reaction the mixture was diluted with 50 ml dichloromethane and extracted three times with 10 ml water each. The organic phase was dried over sodium sulfate and evaporated in vacuum to dryness. 2.28 g (8.7 mmole) (87% theoretical yield) of a mixture of the anomeric **1-O-acetyl-2,3-dideoxy-3-fluoro-5-O-pivaloyl-D-erythro-pentofuranoses** were obtained.

**$^{13}$C-NMR ($CDCl_3$)**

98.9/99.0 (C-1), 39.5 (C-2, J 21.0), 94.1/93.8 (C-3, J 181.1/180.5), 83.7/84.5 (C-4, J 24.9/26.6), 63.6/63.7 (C-5, J 10/10); 27.4/39.1/179.1 (OPiv); 21.6/171.6 (OAc).

**Example 52:**

0.53 g (2 mmol) 1-O-acetyl-2,3-dideoxy-3-fluoro-5-O-pivaloyl-D-erythro-pentofuranose (anomer mixture) were obtained according to Example 51 were introduced in 10 ml absolute dichloromethane saturated at 0 °C with gazeous HCl and agitated until a quantitative reaction has been obtained. After the evaporation in vacuum the **mixture of the anomeric 2,3-dideoxy-3-fluoro-5-O-pivaloyl-D-erythro-pentofuranosyl chlorides** is obtained in a quantitative yield in the form of an oil.

**$^{13}$C-NMR ($CDCl_3$)**

100.5/95.2 (C-1), 45.4/40.0 (C-2, 20.7/20.8), 94.3/93.1 (C-3, 184.8/182.4), 85.0/82.6 ( C-4, 27.7/26.6), 63.9/63.1 ( C-5, 8.7/8.8), 27.3/39.3/179.0 (O-Piv)

**Example 53:**

2.51 g (10.2 mmol) methyl 2,3-dideoxy-3 fluoro-5-O-pivaloyl-D-erythro-pentofuranoside and 4.29 g (15.9 mmol) 5-methyl 2,4-bis-(trimethylsilyloxy)-pyrimidine were dissolved in 100 ml acetonitrile. 3.38 ml (12.7 mmol) trimethylsilyltrifluoromethane sulfonate was added to this solution at room temperature with stirring. After ca. one

hour 30 ml saturated sodium hydrogen carbonate solution was added to the reaction mixture and it was extracted with two lots of 150 ml chloroform. The combined organic phases were washed once with 50 ml water and dried over sodium sulphate, and then evaporated on the rotary evaporator. The residue was purified by column chromatography using silica gel (chloroform;acetone = 9:1). 2.05 g (6.2 mmol) of a **mixture** of **3'-deoxy-3'-fluoro-5'-O-pivaloyl-thymidine and its anomers** were obtained, i.e. a yield 59% of the theoretical.

**alpha/beta-anomer mixture:**
**$^{13}$C-NMR ($CDCl_3$)**
151.4/151.7 (C-1), 165.1/165.3 (C-4), 111.7/112.2 (C-5), 12.8/12.9 ($CH_3$), 135.4/135.9 (C-6), 85.8/87.0 (C-1'), 38.9/39.6 (C-2', J 21.3/20.7), 94.0/94.5 (C-3', J 179.6/178.2), 83.1/85.1 (C-4', J 26.6/24.5), 63.8/64.0 (C-5', J 11.5/10.5)

The mixture was dissolved in 60 ml methanol and 2.14 ml 30% sodium methylate solution in absolute methanol was added to this mixture. After being stirred for 4 hours at room temperature, the mixture was neutralised with acetic acid and taken to dryness on the rotary evaporator, dissolved in chloroform, filtered to free from crystalline sodium acetate and again concentrated to dryness. The oily residue was crystallised from 2-propanol. 0.458 g (2.0 mmol) **3'-deoxy-3'-fluoro-thymidine** was obtained, i.e. 31% of the theoretical yield over the two steps.

**beta-anomer:**
**$^1$H-NMR ($CDCl_3$)**
7.95 (H-6), 1.85 ($CH_3$), 6.18 (H-1', J 7.4, 7.5), 2.4-2.6 (H-2'$_a$, H-2'$_b$, m), 5.33 (H-3', J 2.5, 3.0, 53.5), 4.34 (H-4', J 1.8, 2.6, 27.4), 3.93 (H-5'$_a$, J 1.8, 2.0, 11.8), 3.88 (H-5'$_b$, J 2.6, 11.8)
**$^{13}$C-NMR ($CDCl_3$)**
151.6 (C-2), 165.5 (C-4), 112.3 (C-5), 12.9 ($CH_3$), 139.9 (C-6), 89.3 (C-1'), 38.4 (C-2', J 20.6), 95.8 (C-3', J 177.8), 86.7 (C-4', J 24.9), 63.4 (C-5', J 10.6)

**Example 54:**

**2',3'-dideoxy-3'-fluoro-uridine** was obtained by the method described in Example 53 using 2,4-bis(trimethylsilyloxy)pyrimidine as starting material. The yield was 36% of the theoretical.

**beta-anomer:**
**$^1$H-NMR (DMSO-d$_6$)**
Delta 6.21 (H-1', J 5.5, 8.9), 2.1-2.6 (H-2'a, H-2'b, m) 5.31 (H-3', J 4.5, 53.5), 4.18 (H-4', J 3.0, 4.0, 27.5), 3.64 (H-5'a, J 3.0, 11.9). 3.58 (H-5'$_b$, J 4.0, 11.9), 5.69 (H-5, J 8.2), 7.88 (H-6, J 8.2)
**$^{13}$C-NMR (DMSO-d$_6$)**
152.1 (C-2), 165.2 (C-4), 103.2 (C-5), 141.7 (C-6), 84.9 (C-1'), 37.6 (C-2', J 20.3), 95.9 (C-3', J 174.8), 86.5 (C-4', J 22.9), 61.4 (C-5', J 11.2)

**Example 55:**

**2',3'-dideoxy-3',5-difluoro-uridine** was obtained by the method described in Example 53 using 5-fluoro-2,4-bis(trimethylsilyloxy)pyrimidine as starting material. The yield was 30% of the theoretical.

**alpha-anomer:**
**$^1$H-NMR (DMSO-d$_6$)**
6.31 (H-1', J 1,6), 2.78 (H-2'$_a$, J 5, 7, 16, 42), 2.50 (H-2'$_b$, J 16, 24), 5.24 (H-3', J 5,54), 4.85 (H-4', J 4, 5, 24), 4.17 (H-5'$_a$, J 4, 7), 4.08 (H-5'$_b$, J 4, 7), 7.54 (H-6, J 6)
**$^{13}$C-NMR (DMSO-d$_6$)**
150.3 (C-2), 158.0 (C-4, J 26.3), 141.3 (C-5, J 231.5), 125.5 (C-6, J 34.7), 87.2 (C-1'), 38.5 (C-2', J 20.3), 94.9 (C-3', J 175.4), 84.7 (C-4', J 24.8), 63.8 (C-5', J 11.7)

**beta-anomer:**
**$^1$H-NMR (DMSO-d$_6$)**
6.20 (H-1', J 1.8, 5.6, 8.8), 2.47 (H-2'$_a$, J 5.6, 14.7, 22.9), 2.27 (H-2'$_b$, J 4.8, 9.0, 14.7, 40.2), 5.31 (H-3', J 4.6, 53.7), 4.20 (H-4', J 3.5, 3.5, 27.2), 3.66 (H-5'$_a$, J 3.5, 11.9), 3.61 (H-5'b, J 3.5, 11.9), 8.10 (H-6 J 6.2)
**$^{13}$C-NMR (DMSO-d$_6$)**
150.6 (C-2), 158.6 (C-4. J 26.4), 141.5 (C-5, J 231.7), 125.7 (C-6, J 34.8). 85.2 (C-1'), 37.6 (C-2', J 20.3), 95.8 (C-3', J 174.8), 85.9 (C-4', J 23.0), 61.3 (C-5', J 11.2)

**Example 56:**

**5-chloro-2',3'-dideoxy-3'-fluoro-uridine** was obtained by the method described in Example 53 using 5-chloro-2,4-bis(trimethylsilyloxy)pyrimidine as starting material. The yield was 34% of the theoretical.

**alpha-anomer:**
**$^1$H-NMR (DMSO-d$_6$)**
6.15 (H-1', J 1.2, 6.1), 2.72 (H-2'$_a$, J 5.1, 7.4, 15.8, 42.9), 2.38 (H-2'$_b$, J 15.8, 24.1), 5.33 (H-3', J 4.6, 54.3), 4.71 (H-4', J 3.4, 4.9, 24.4), 3.51 (H-5'$_a$, J 3.4, 12.5), 3.43 (H-5'$_b$, J 4.9, 12.5), 7.90 (H-6)
**$^{13}$C-NMR (DMSO-d$_6$)**
151.0 (C-2), 160.8 (C-4), 107.9 (C-5), 138.6 (C-6), 87.5 (C-1'), 38.9 (C-2', J 20.0), 95.6 (C-3', J 173.6), 88.3 (C-4', J 21.6), 61.4 (C-5', J 11.3)

**beta-anomer:**
**$^1$H-NMR (DMSO-d$_6$)**

6.20 (H-1' J 5.8, 8.9), 2.48 (H-2'$_a$, J 5.8, 14.5, 21,7), 2.32 (H-2'$_b$, J 4.9, 8.9, 14.5, 40.2), 5.32 (H-3', J 4.6, 53.7), 4.22 (H-4', J 3.3, 3.3, 23.2), 3.65 (H-5'$_a$, H-5'$_b$, bs), 8.15 (H-6)

**$^{13}$C-NMR (DMSO-d$_6$)**

151.1 (C-2), 160.6 (C-4), 108.7 (C-5), 138.9 (C-6), 85.5 (C-1'), 38.0 (C-2', J 20.5), 95.8 (C-3', J 175.0), 86.1 (C-4', J 23.0), 61.3 (C-5', J 11.1)

**Example 57:**

To a solution of 2,32 g (10 mmol) methyl 2-deoxy-5-O-pivaloyl-alpha-D-threo-pentofuranoside in 25 ml absolute dichloromethane and 3 ml absolute pyridine a mixture of 2 ml (3.35 g, 12 mmol) trifluoromethanesulfonic acid anhydride and 10 ml absolute dichloromethane was dropped under agitation at -30° C. After warming to room temperature the reaction mixture was extracted with diluted hydrochloric acid, sodium hydrogenocarbonate solution and water. The organic phase was dried over sodium sulfate, separated from the drying agent by filtration and evaporated on the rotary evaporator to a syrup. Thereafter, the syrup was taken up in 30 ml acetonitrile, cooled to -25° C and 5.0 g (18.6 mmol) tetrabutylammonium cyanide were added. After a reaction time of 2 hours at approximately 0° C, the solvent was evaporated on the rotary evaporator and **methyl 3-cyano-2,3-dideoxy-5-O-pivaloyl-α-D-erythro-pentofuranoside** (1.07 g. 44 % theoretical yield) was isolated by column chromatography (petroleum ether : ethyl acetate 2 : 1).

**$^1$H-NMR (CDCl$_3$)**

5.11 (H-1, J 1.5, 4.9), 2.46 (H-2$_a$, J 4.9, 10.6, 13.5), 2.25 (H-2$_b$, J 1.5, 5.3, 13.5), 2.94 (H-3, J 5.3, 7.0, 10.6), 4.45 (H-4, J 4.6, 4.6, 7.0), 4.26 (H-5$_a$, H-5$_b$,J 4.6); 3.38 ($OCH_3$), 1.21 (3 $CH_3$).

**$^{13}$C-NMR (CDCl$_3$)**

104.9 (C-1), 37.1 (C-2), 29.1 (C-3), 78.0 (C-4), 63.4 (C-5); 55.1 ($OCH_3$); 120.1 (CN); 27.0/38.8/178.6 (OPiv).

**Example 58:**

Methyl-3-cyano-2,3-dideoxy-5-O-pivaloyl-α-D-erythro-pentofuranoside, is, as disclosed in Example 49, reacted to **methyl 3-cyano-2,3-dideoxy-a-D-erythro-pentofuranoside** (yield: 91%).

**$^1$H-NMR (CDCl$_3$)**

5.13 (H-1, J 1.6, 5.0), 2.46 (H-2$_a$, J 5.0, 10.7, 13.5), 2.24 (H-2$_b$, J 1.6, 5.6, 13.5), 3.13 (H-3, J 5.6. 7.3, 10.8), 4.32 (H-4, J 3.0, 3.0, 7.3), 3.90 (H-5$_a$), J 3.0, 3.0, 12.5), 3.72 (H-5$_b$, J 3.0, 7.5, 12.5), 2.59 (OH, J 3.0,7.5); 3.38 ($OCH_3$)

**13C-NMR (CDCl3)**

105.1 (C-1), 37.3 (C-2), 27.3 (C-3), 80.7 (C-4), 61.5 (C-5); 120.7 (CN); 55.1 ($OCH_3$).

**Example 59:**

Methyl-2-deoxy-5-O-pivaloyl-α-D-threo-pentofuranoside was reacted to the 3-triflate as disclosed in Example 57. 7.3 g (20 mmol) of the sirupy product were thereafter taken up in 50 ml N,N-dimethylformamide, cooled to -30° C and reacted under agitation with 5.0 (50 mmol) potassium thiocyanate added in portions. After an hour the solvent was evaporated under oil pump vacuum, the residue was distributed between water (30 ml) and dichloromethane (100 ml), whereafter the organic phase was dried over sodium sulfate, filtered and evaporated to dryness. The purification by column chromatography (petroleum ether: ethyl acetate 5: 1) provided 1.5 g (27% theoretical yield) of **methyl 2-deoxy-5-O-pivaloyl-3-thiocyanato-α-D-erythro-pentofuranoside.**

**$^1$H-NMR (CDCl$_3$)**

5.12 (H-1, J 1.3, 5.0), 2.62 (H-2$_a$, J 5.0, 9.6, 14.3), 2.10 (H-2$_b$, J 1.3, 4.2, 14.3), 3.62 (H-3, J 4.2, 5.6. 9.6), 4.15 - 4.40 (H-4, H-5$_a$, H-5$_b$, m); 3.37 ($OCH_3$), 1.21 (3 $CH_3$)

**$^{13}$C-NMR (CDCl$_3$)**

104.5 (C-1), 39.9 (C-2), 45.1 (C-3), 82.0 (C-4), 63.3 (C-5); 55.1 ($OCH_3$); 111.6 (SCN); 27.1/38.9/178.8 (Piv).

**Example 55:**

2.34 g (10 mmol) methyl 2,3-dideoxy-3-fluoro-5-O-pivaloyl-α-D-erythro-pentofuranoside obtained according to Example 42 are dissolved in a mixture of 25 ml acetic acid and 5 ml acetic acid anhydride and added under cooling with ice and agitation slowly with 0.2 ml concentrated sulphuric acid. After the quantitative reaction the reaction product is diluted with 50 ml dichloromethane and extracted three times with 10 ml water each. The organic phase is after having been dried over sodium sulfate evaporated in vacuum to dryness. 2.28 g (8.7 mmol) (87% theoretical yield) of a **mixture** of the **1-O-acetyl-2,3-dideoxy-3-fluoro-5-O-pivaloyl-α-D-erythro-pentofuranose anomers** is obtained.

**$^{13}$C-NMR (CDCl$_3$)**

89.9/99.0 (C-1), 39.5 (21.0 Hz), C-2), 94.1/93.8 (181.1/180.5 Hz, C-3), 83.7/84.5 (24.9/26.6 Hz, C-4), 63.6/63.7 (10/10 Hz, C-5), 27.4/39.1/179.1 (O-Piv), 21.6/171.6 (O-Ac)

**Claims**

1. A process for the manufacture of 2-deoxy-D-threo-pentofuranosides of formula 1

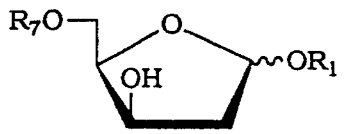

(1)

wherein $R_1$ is an alkyl group having 1-4 carbon atoms and $R_7$ is a hydroxy protection group, **characterized in that** a 2-deoxy-D-erythro-pentofuranoside of formula 2

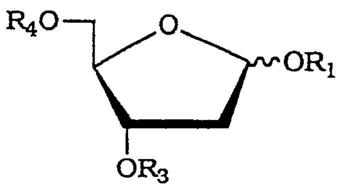

(2)

wherein $R_1$ is an alkyl group having 1 to 4 carbon atoms, $R_3$ is an alkyl-, aryl-, alkylaryl- or aralkyl-sulfonyl group, either unsubstituted or substituted one or more times by halogen atoms, nitro or alkoxy groups, an imidazolesulfonyl or halosulfonyl group, and $R_4$ has the same meaning as $R_3$ or signifies a hydroxy protection group,
is reacted either:

a) if $R_4$ in formula 2 has the same meaning as $R_3$, with at least two equivalents of an ionogenic nitrite in a dilution solvent to give a compound of formula 3

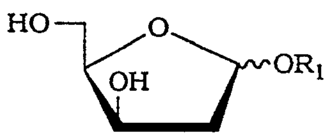

(3)

wherein $R_1$ is an alkyl group having 1 to 4 carbon atoms, after which position 5 is protected with a hydroxy protection group $R_4$ by methods known per se, or

b) if $R_4$ in formula 2 has the same meaning as $R_3$, with 1-1.5 equivalents of a nucleophilic carboxylate or benzylate to give a compound of formula 4

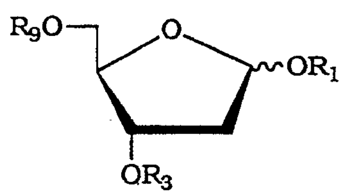

(4)

wherein $R_1$ and $R_3$ retain their meanings and $R_9$ is an acyl or benzyl group, after which at least a molar amount of an ionogenic nitrite in a dilution solvent is added, giving rise to a compound of formula 5

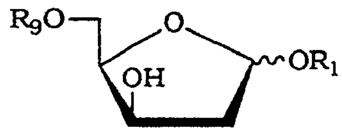

(5)

wherein $R_1$ and $R_9$ retain their meanings, or

c) if $R_4$ in formula 2 has the same meaning as $R_3$, with at least two equivalents of a nucleophilic carboxylate to give a compound of formula 6

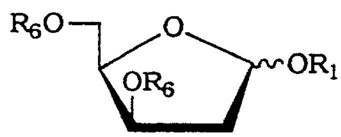

(6)

wherein $R_1$ retains its meaning and $R_6$ is an acyl group, after which $R_6$ is cleaved in the usual way and then position 5 is protected by a hydroxy protection group $R_4$ by methods known per se, or

d) if $R_4$ in formula 2 is a hydroxy protection group, with at least an equimolar amount of a nucleophilic carboxylate or an ionogenic nitrite in a dilution solvent to give a compound of formula 7

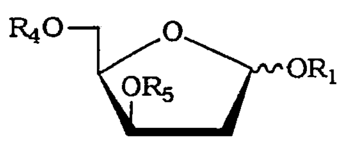

(7)

wherein $R_1$ and $R_4$ retain their meanings and $R_5$ is a hydrogen atom or an acyl group, after which $R_5$ is cleaved in the usual way if it is not already hydrogen.

2. The process according to claim 1, **characterized in that** a compound of formula 2 is used wherein the hydroxy protection group $R_4$ is an alkanoyl, aroyl, 4-phenylaroyl, alkyloxycarbonyl, aryloxycarbonyl, aralkyl, alkyl-, aryl- or alkylaryl-silyl, tetrahydrofuranyl or tetrahydropyranyl group, which groups are either unsubstituted or substituted.

3. The process according to claim 1, **characterized in that** as said ionogenic nitrite compound an alkali or a tetraalkyl- or trialkylaralkylammonium nitrite, preferably sodium or potassium nitrite is used.

**4.** A process for the manufacture of 3-substituted 2-deoxy-D-erythro-pentofuranose derivatives of formula 8

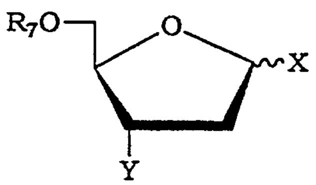

(8)

wherein $R_7$ is hydrogen or a hydroxy protection group, X is halogen or a group $OR_{10}$ wherein $R_{10}$ is hydrogen, an alkyl group having 1 to 4 carbon atoms or an acyl group and Y is halogen, an azido, cyano or thiocyanato group, **characterized in that** a 2-deoxy-D-threo-pentofuranoside of formula 1

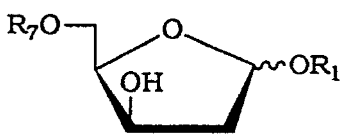

(1)

wherein $R_1$ is an alkyl group having 1 to 4 carbon atoms and $R_7$ is a hydroxy protection group, as obtained with the process according to claims 1 to 3, is converted by the addition of at least 1 mole of an alkyl-, aryl-, alkylaryl- or aralkyl-sulfonic acid halide or anhydride, either unsubstituted or substituted one or more times by halogen atoms, nitro or alkoxy groups, in the presence of a base or by addition of a 1-2 molar amount of sodium hydride followed by N,N'-sulfuryl-diimidazole or by reaction with an at least molar amount of sulfuryl chloride in the presence of imidazole or in the presence of a tertiary amine with or without a dilution solvent that is inert under the reaction conditions to a compound of formula 9

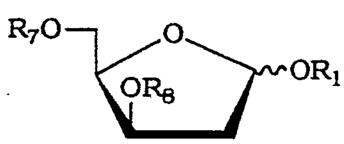

(9)

wherein $R_1$ and $R_7$ retain their meanings and $R_8$ is an alkyl-, aryl-, alkylaryl- or aralkyl-sulfonyl group that is either unsubstituted or substituted one or more times by halogen atoms, nitro or alkoxy groups, or signifies an imidazolesulfonyl or halosulfonyl group, after which the compound of formula 9 is converted by the addition of at least a molar amount of an ionogenic halide, azide, cyanide or thiocyanate compound or by reaction of the compound of formula 1 with at least an equivalent amount of a sulfurtrifluoride of a secondary amine in a dilution solvent that is inert under the reaction conditions to a compound of formula 10

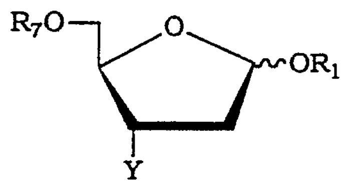

(10)

wherein $R_1$, $R_7$ and Y retain their meanings, whereafter said compound of formula 10, is transformed into a compound of formula 11 by methods known per se,

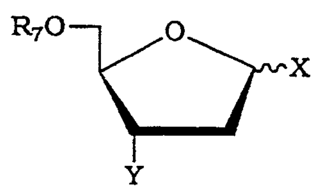

(11)

wherein Y and $R_7$ are as defined above, and X is $OR_{10}$ wherein $R_{10}$ is an acyl group or hydrogen, and, if desired, said compound of formula 11 wherein X is $OR_{10}$ is reacted by methods known per se to provide a compound of formula 11 wherein Y and $R_7$ are as defined above, and X is halogen.

**5.** The process according to claim 4, **characterized in that** for the manufacture of the compounds of formula 10

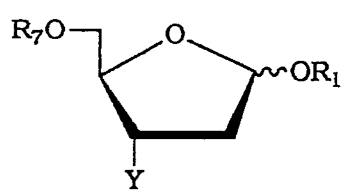

(10)

wherein $R_1$ is an alkyl group having 1 to 4 carbon atoms, a compound of formula 1 is used wherein the hydroxy protection group $R_7$

is an alkanoyl, aroyl, alkyloxycarbonyl, aryloxycarbonyl, aralkyl, alkyl-, aryl- or alkylaryl-silyl, tetrahydrofuranyl or tetrahydropyranyl group, which groups are either unsubstituted or substituted, and that for the manufacture of the compounds of formula 11 a compound of formula 10 is used wherein the hydroxy protection group $R_7$ is an alkanoyl, aroyl, alkyloxycarbonyl or aryloxycarbonyl group, which groups are either unsubstituted or substituted, or the benzyl group, preferably with an acetyl, triphenylmethyl, 4-phenylbenzoyl, pivaloyl, isobutyloxycarbonyl or benzoyl group.

**6.** The process for the manufacture of 2-deoxy-D-threo-pentofuranosides of formula 1 according to claims 1 to 3, **characterized in that** the 2-deoxy-D-erythro-pentofuranoside of formula 2 is manufactured by converting 2-deoxy-D-erythro-pentose of the formula 12

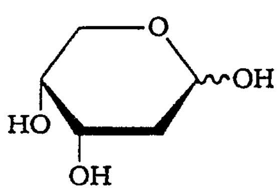

(12)

to a glycoside using an alkanol of formula $R_1OH$, wherein $R_1$ is an alkyl group having 1 to 4 carbon atoms to provide an alkyl 2-deoxy-D-erythro-pentofuranoside of formula 13

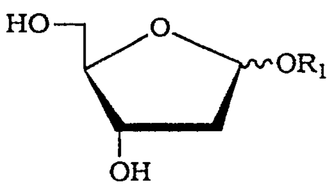

(13)

wherein $R_1$ is an alkyl group having 1 to 4 carbon atoms, then protecting position 5 by a hydroxy protection group $R_4$ by methods known per se to provide an alkyl 2-deoxy-D-erythro-pentofuranoside of formula 14

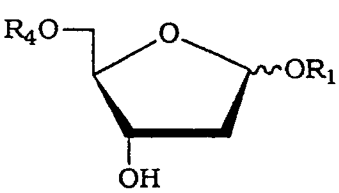

(14)

wherein $R_1$ is as defined above and $R_4$ is a hydroxy protection group, and then sulfonating said compound of formula 14 with 1 - 1.5 equivalents of an alkyl-, aryl-, alkylaryl- or aralkyl-sulfonic acid halide or anhydride, either unsubstituted or substituted one or more times by halogen atoms, nitro or alkoxy groups, in the presence of a base or by addition of a 1-2 molar amount of sodium hydride followed by N,N'-sulfuryl-diimidazole or by reaction with an at least molar amount of sulfuryl chloride in the presence of imidazole or in the presence of a tertiary amine with a dilution solvent that is inert under the reaction conditions.

**7.** The process according to claim 6, **characterized in that** as said position 5 is protected with an alkanoyl, aroyl, 4-phenylaroyl, alkyloxycarbonyl, aryloxycarbonyl, aralkyl, triphenylmethyl, alkyl-, aryl- or alkylarylsilyl, tetrahydrofuranyl or tetrahydropyranyl group, which groups are either unsubstituted or substituted, preferably with an acetyl, triphenylmethyl, pivaloyl, isobutyloxycarbonyl, tetrahydropyranyl, 4-phenyl benzoyl or benzoyl group.

**8.** 2-Deoxy-D-pentofuranose derivatives of the general formula 15

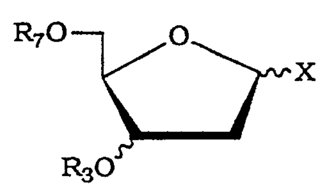

(15)

wherein $R_7$ is an alkanoyl, aroyl, 4-phenylaroyl, alkyloxycarbonyl, aryloxycarbonyl, aralkyl, triphenylmethyl, alkyl-, aryl- or alkylaryl-silyl, tetrahydrofuranyl or tetrahydropyranyl group, which groups are either unsubstituted or substituted, preferably a pivaloyl, tert.-butyldimethylsilyl, isobutyloxycarbonyl or tetrahydropyranyl group and $R_3$ is hydrogen or an alkyl-, aryl-, alkylaryl- or aralkyl-sulfonyl group, either unsubstituted or substituted one or more times by halogen atoms, nitro or alkoxy groups, or an imidazolsulfonyl or halosulfonyl group. and X is a halogen atom or $OR_1$ wherein $R_1$ is an alkyl group having 1 to 4 carbon atoms or an acyl group, the threo and erythro derivatives and the $\alpha$- and $\beta$-anomers thereof, with the exclusion of methyl 2-deoxy-5-O-triphenylmethyl-D-erythro-pentofuranoside $\alpha$ and $\beta$ anomer, methyl 5-O-benzoyl-2-deoxy-D-erythro-pentofuranoside $\alpha/\beta$ mixture, methyl 5-O-tert.-butyldimethylsilyl-2-deoxy-D-erythro-pentofuranoside $\alpha/\beta$ mixture, methyl 2-deoxy-3-O-methanesulfonyl-5-O-pivaloyl-D-erythro-pentofuranoside α anomer, methyl 2-deoxy-5-O-(4-phenylbenzoyl)-D-erythro-pentofuranoside α and β anomer, methyl 2-deoxy-5-O-triphenylmethyl-D-threo-pentofuranoside α and β anomer, methyl 2-deoxy-5-O-(4-methylbenzoyl)-D-threo-pentofuranoside α/β mixture, methyl 5-O-tert.-butyldimethylsilyl-2-deoxy-D-threo-pentofuranoside β anomer, methyl 2-deoxy-5-O-(4-phenylbenzoyl)-D-threo-pentofuranoside β anomer, methyl 5-O-tert.-butyldiphenylsilyl-2-deoxy-D-threo-pentofuranoside α and β anomer, methyl 5-O-tert.-butyldiphenylsilyl-2-deoxy-3-O-trifluoromethanesulfonyl-D-threo-pentofuranoside α and β anomer and methyl 2-deoxy-D-threo-pentofuranoside α/β mixture.

**9.** 2-Deoxy-D-erythro-pentofuranosides of the general formula 2

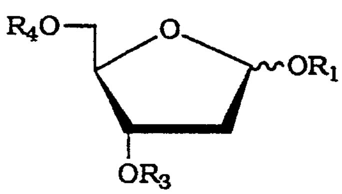

(2)

wherein R4 is an alkanoyl, aroyl, 4-phenylaroyl, alkyloxycarbonyl, aryloxycarbonyl, aralkyl, triphenylmethyl, alkyl-, aryl- or alkylaryl-silyl, tetrahydrofuranyl or tetrahydropyranyl group, which groups are either unsubstituted or substituted, preferably a pivaloyl, benzyl, 4-methylbenzoyl, tert.-butyldimethylsilyl, isobutyloxycarbonyl or tetrahydropyranyl group, $R_3$ is hydrogen or an alkyl-, aryl-, alkylaryl- or aralkyl-sulfonyl group, either unsubstituted or substituted one or more times by halogen atoms, nitro or alkoxy groups, or an imidazolesulfonyl or halosulfonyl group, and $R_1$ is an alkyl group having 1 to 4 carbon atoms and the α- and β-anomers thereof, with the exclusion of methyl 2-deoxy-5-O-triphenylmethyl-D-erythro-pentofuranoside α and β anomer, methyl 5-O-benzoyl-2-deoxy-D-erythro-pentofuranoside α/β mixture, methyl 5-O-tert.-butyldimethylsilyl-2-deoxy-D-erythro-pentofuranoside α/β mixture, methyl 2-deoxy-3-O-methanesulfonyl-5-O-pivaloyl-D-erythro-pentofuranoside α anomer and methyl 2-deoxy-5-O-(4-phenylbenzoyl)-D-erythro-pentofuranoside α and β anomer.

**10.** 2-Deoxy-D-threo-pentofuranosides of the general formula 9

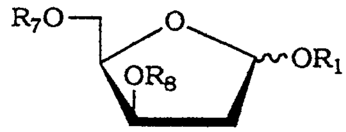

(9)

wherein $R_7$ is an alkanoyl, aroyl, 4-phenylaroyl, alkyloxycarbonyl, aryloxycarbonyl, aralkyl, triphenylmethyl, alkyl-, aryl- or alkylaryl-silyl, tetrahydrofuranyl or tetrahydropyranyl group, which groups are either unsubstituted or substituted, preferably a pivaloyl, benzyl, tert.-butyldimethylsilyl, isobutyloxycarbonyl or tetrahydropyranyl group, $R_8$ is hydrogen or an alkyl-, aryl-, alkylaryl- or aralkyl-sulfonyl group, either unsubstituted or substituted one or more times by halogen atoms, nitro or alkoxy groups, a benzoyl or acetyl group, an imidazolesulfonyl or halosulfonyl group, and $R_1$ is an alkyl group having 1 to 4 carbon atoms, and the α- and β-anomers thereof, with the exclusion of methyl 2-deoxy-5-O-triphenylmethyl-D-threo-pentofuranoside α and β anomer, methyl 2-deoxy-5-O-(4-methyl-benzoyl)-D-threo-pentofuranoside α/β mixture, methyl 5-O-tert.-butyldimethylsilyl-2-deoxy-D-threo-pentofuranoside β anomer, methyl 2-deoxy-5-O-(4-phenylbenzoyl)-D-threo-pentofuranoside β anomer, methyl 5-O-tert.-butyldiphenylsilyl-2-deoxy-D-threo-pentofuranoside α and β anomer, methyl 5-O-tert.-butyldiphenylsilyl-2-deoxy-3-O-trifluoromethanesulfonyl-D-threo-pentofuranoside α and β anomer methyl 2-deoxy-D-threo-pentofuranosideα/β mixture and methyl 3-O-acetyl-5-O-benzyl-2-deoxy-D-threo-pentofuranoside α/β anomer mixture.

**11.** 2-Deoxy-D-erythro-pentofuranoside derivatives of the general formula 10

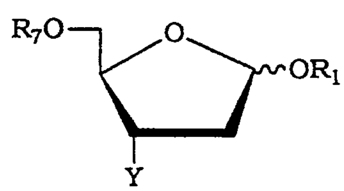

(10)

wherein $R_7$ is a pivaloyl, triphenylmethyl, tert.-butyldimethylsilyl, isobutyloxycarbonyl or tetrahydropyranyl group, Y is halogen, an azido, cyano or thiocyanato group and $R_1$ is an alkyl group having 1 to 4 carbon atoms, and the α- and β-anomers thereof.

**12.** 2-Deoxy-D-erythro-pentofuranoside derivatives of the general formula 11

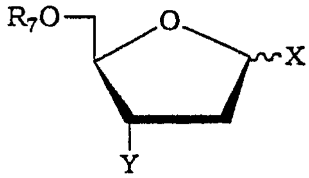

(11)

wherein $R_7$ is a pivaloyl, triphenylmethyl, tert.-butyldimethylsilyl, isobutyloxycarbonyl or tetrahydropyranyl group, Y is halogen, an azido, cyano or thiocyanato group and X is halogen or a group $OR_{10}$ wherein $R_{10}$ is hydrogen or an acyl group, and the $\alpha$- and $\beta$-anomers thereof.

**13.** Methyl 2-deoxy-5-O-pivaloyl-D-erythro-pentofuranoside.

**14.** Methyl 2-deoxy-3-O-methanesulfonyl-5-O-pivaloyl-D-erythro-pentofuranoside.

**15.** Methyl 2-deoxy-5-O-pivaloyl-D-threo-pentofuranoside.

**16.** Methyl 2,3-dideoxy-3-fluoro-5-O-pivaloyl-D-erythro-pentofuranoside and the $\alpha$- and $\beta$-anomers thereof.

**17.** 2,3-Dideoxy-3-fluoro-D-erythro-pentose.

**18.** The use of the compounds of formulas 8 or 10 as defined and manufactured according to claim 4 and the compounds of formula 11 as defined in claim 12, for the manufacture of 2'-deoxynucleosides of the following general formula 16

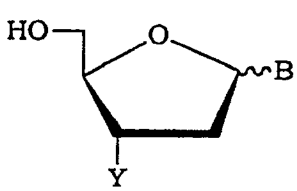

(16)

wherein Y is halogen, an azido, cyano or thiocyanato group and B is a N-heterocyclic base residue, **characterized in that** a compound of formula 10 or 11, wherein X is halogen or a group $OR_{10}$, wherein $R_{10}$ is an acyl group, is condensed with a N-heterocyclic base using methods known per se, whereafter any protective groups present are cleaved off and, if desired, the anomers of the compounds of formula 16 are separated by crystallisation or chromatography using methods known per se.

**19.** The use according to claim 18, **characterized in that** a compound of formula 10 wherein $R_1$ is a methyl group and $R_7$ is an acyl group is condensed with at least an equimolar amount of a silylated purine or pyrimidine base under catalysis, the protective groups present are cleaved off and the anomers of formula 16 formed are separated by crystallisation or chromatography using methods known per se.

**Claims for the following Contracting State: ES**

**1.** A process for the manufacture of 2-deoxy-D-threo-pentofuranosides of formula 1

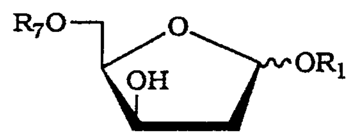

(1)

wherein $R_1$ is an alkyl group having 1-4 carbon atoms and $R_7$ is a hydroxy protection group, **characterized in that** a 2-deoxy-D-erythro-pentofuranoside of formula 2

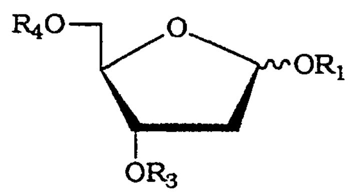

(2)

wherein $R_1$ is an alkyl group having 1 to 4 carbon atoms, $R_3$ is an alkyl-, aryl-, alkylaryl- or aralkyl-sulfonyl group, either unsubstituted or substituted one or more times by halogen atoms, nitro or alkoxy groups, an imidazolesulfonyl or halosulfonyl group, and $R_4$ has the same meaning as $R_3$ or signifies a hydroxy protection group,
is reacted either:

a) if $R_4$ in formula 2 has the same meaning as $R_3$, with at least two equivalents of an ionogenic nitrite in a dilution solvent to give a compound of formula 3

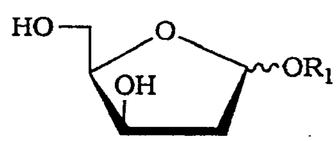

(3)

wherein $R_1$ is an alkyl group having 1 to 4 carbon atoms, after which position 5 is protected with a hydroxy protection group $R_4$

by methods known per se, or

b) if $R_4$ in formula 2 has the same meaning as $R_3$, with 1 - 1.5 equivalents of a nucleophilic carboxylate or benzylate to give a compound of formula 4

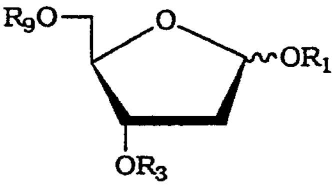

(4)

wherein $R_1$ and $R_3$ retain their meanings and $R_9$ is an acyl or benzyl group, after which at least a molar amount of an ionogenic nitrite in a dilution solvent is added, giving rise to a compound of formula 5

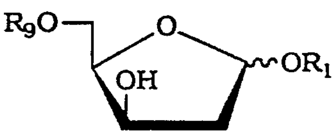

(5)

wherein $R_1$ and $R_9$ retain their meanings, or

c) if $R_4$ in formula 2 has the same meaning as $R_3$, with at least two equivalents of a nucleophilic carboxylate to give a compound of formula 6

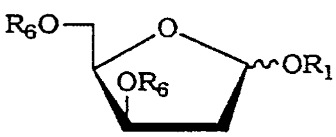

(6)

wherein $R_1$ retains its meaning and $R_6$ is an acyl group, after which $R_6$ is cleaved in the usual way and then position 5 is protected by a hydroxy protection group $R_4$ by methods known per se, or

d) if $R_4$ in formula 2 is a hydroxy protection group, with at least an equimolar amount of a nucleophilic carboxylate or an ionogenic nitrite in a dilution solvent to give a compound of formula 7

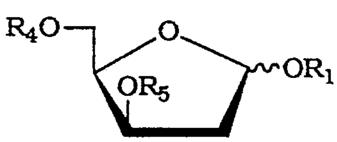

(7)

wherein $R_1$ and $R_4$ retain their meanings and $R_5$ is a hydrogen atom or an acyl group, after which $R_5$ is cleaved in the usual way if it is not already hydrogen.

2. The process according to claim 1, **characterized in that** a compound of formula 2 is used wherein the hydroxy protection group $R_4$ is an alkanoyl, aroyl, 4-phenylaroyl, alkyloxycarbonyl, aryloxycarbonyl, aralkyl, alkyl-, aryl- or alkylaryl-silyl, tetrahydrofuranyl or tetrahydropyranyl group, which groups are either unsubstituted or substituted.

3. The process according to claim 1, **characterized in that** as said ionogenic nitrite compound an alkali or a tetraalkyl- or trialkylaralkylammonium nitrite, preferably sodium or potassium nitrite is used.

4. A process for the manufacture of 3-substituted 2-deoxy-D-erythro-pentofuranose derivatives of formula 8

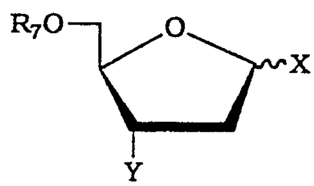

(8)

wherein $R_7$ is hydrogen or a hydroxy protection group, X is halogen or a group $OR_{10}$ wherein $R_{10}$ is hydrogen, an alkyl group having 1 to 4 carbon atoms or an acyl group and Y is halogen, an azido, cyano or thiocyanato group, **characterized in that** a 2-deoxy-D-threo-pentofuranoside of formula 1

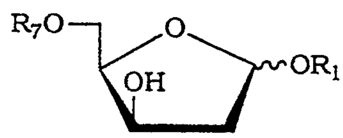

(1)

wherein $R_1$ is an alkyl group having 1 to 4 carbon atoms and $R_7$ is a hydroxy protection group, as obtained with the process according to claims 1 to 3, is converted by the addition of at least 1 mole of an alkyl-, aryl-, alkylaryl- or aralkyl-sulfonic acid halide or anhydride, either unsubstituted or substituted one or more times by halogen atoms, nitro or alkoxy groups, in the presence of a base or by addition of a 1-2 molar amount of sodium hydride followed by N,N'-sulfuryl-diimidazole or by reaction with an at least molar amount of sulfuryl chloride in the presence of imidazole or in the presence of a

tertiary amine with or without a dilution solvent that is inert under the reaction conditions to a compound of formula 9

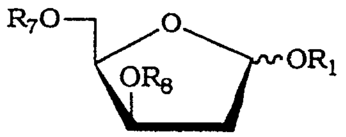

(9)

wherein $R_1$ and $R_7$ retain their meanings and $R_8$ is an alkyl-, aryl-, alkylaryl- or aralkyl-sulfonyl group that is either unsubstituted or substituted one or more times by halogen atoms, nitro or alkoxy groups, or signifies an imidazolesulfonyl or halosulfonyl group, after which the compound of formula 9 is converted by the addition of at least a molar amount of an ionogenic halide, azide, cyanide or thiocyanate compound or by reaction of the compound of formula 1 with at least an equivalent amount of a sulfurtrifluoride of a secondary amine in a dilution solvent that is inert under the reaction conditions to a compound of formula 10

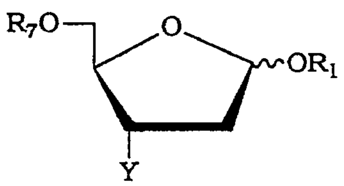

(10)

wherein $R_1$, $R_7$ and Y retain their meanings, whereafter said compound of formula 10, is transformed into a compound of formula 11 by methods known per se,

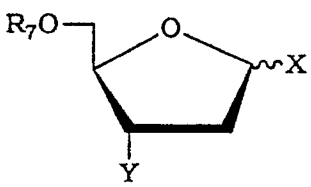

(11)

wherein Y and $R_7$ are as defined above, and X is $OR_{10}$ wherein $R_{10}$ is an acyl group or hydrogen, and, if desired, said compound of formula 11 wherein X is $OR_{10}$ is reacted by methods known per se to provide a compound of formula 11 wherein Y and $R_7$ are as defined above, and X is halogen.

**5.** The process according to claim 4, **characterized in that** for the manufacture of the compounds of formula 10

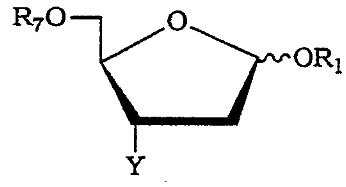

(10)

wherein $R_1$ is an alkyl group having 1 to 4 carbon atoms, a compound of formula 1 is used wherein the hydroxy protection group $R_7$ is an alkanoyl, aroyl, alkyloxycarbonyl, aryloxycarbonyl, aralkyl, alkyl-, aryl- or alkylaryl-silyl, tetrahydrofuranyl or tetrahydropyranyl group, which groups are either unsubstituted or substituted, and that for the manufacture of the compounds of formula 11 a compound of formula 10 is used wherein the hydroxy protection group $R_7$ is an alkanoyl, aroyl, alkyloxycarbonyl or aryloxycarbonyl group, which groups are either unsubstituted or substituted, or the benzyl group, preferably with an acetyl, triphenylmethyl, 4-phenylbenzoyl, pivaloyl, isobutyloxycarbonyl or benzoyl group.

**6.** The process for the manufacture of 2-deoxy-D-threo-pentofuranosides of formula 1 according to claims 1 to 3, **characterized in that** the 2-deoxy-D-erythro-pentofuranoside of formula 2 is manufactured by converting 2-deoxy-D-erythro-pentose of the formula 12

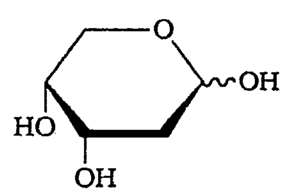

(12)

to a glycoside using an alkanol of formula $R_1OH$, wherein $R_1$ is an alkyl group having 1 to 4 carbon atoms to provide an alkyl 2-deoxy-D-erythro-pentofuranoside of formula 13

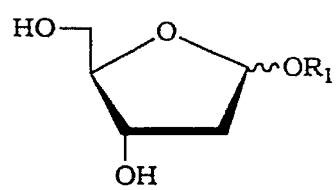

(13)

wherein $R_1$ is an alkyl group having 1 to 4 carbon atoms, then protecting position 5 by a hydroxy protection group $R_4$ by methods known per se to provide an alkyl 2-deoxy-D-erythro-pentofuranoside of formula 14

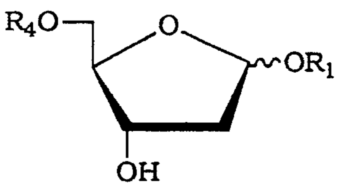

(14)

wherein $R_1$ is as defined above and $R_4$ is a hydroxy protection group, and then sulfonating said compound of formula 14 with 1 - 1.5 equivalents of an alkyl-, aryl-, alkylaryl- or aralkyl-sulfonic acid halide or anhydride, either unsubstituted or substituted one or more times by halogen atoms, nitro or alkoxy groups, in the presence of a base or by addition of a 1-2 molar amount of sodium hydride followed by N,N'-sulfuryl-diimidazole or by reaction with an at least molar amount of sulfuryl chloride in the presence of imidazole or in the presence of a tertiary amine with a dilution solvent that is inert under the reaction conditions.

7. The process according to claim 6, **characterized in that** as said position 5 is protected with an alkanoyl, aroyl, 4-phenylaroyl, alkyloxycarbonyl, aryloxycarbonyl, aralkyl, triphenylmethyl, alkyl-, aryl- or alkylaryl silyl, tetrahydrofuranyl or tetrahydropyranyl group, which groups are either unsubstituted or substituted, preferably with an acetyl, triphenylmethyl, pivaloyl, isobutyloxycarbonyl, tetrahydropyranyl, 4-phenyl benzoyl or benzoyl group.

8. The use of the compounds of formula 8 or 10 as defined and manufactured according to claim 4, for the manufacture of 2'-deoxynucleosides of the following general formula 16

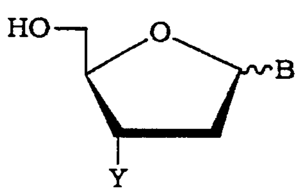

(16)

wherein Y is halogen, an azido, cyano or thiocyanato group and B is a N-heterocyclic base residue, **characterized in that** a compound of formula 10 or 11, wherein X is halogen or a group $OR_{10}$, wherein $R_{10}$ is an acyl group, is condensed with a N-heterocyclic base using methods known per se, whereafter any protective groups present are cleaved off and, if desired, the anomers of the compounds of formula 16 are separated by crystallisation or chromatography using methods known per se.

9. The use according to claim 8, **characterized in that** a compound of formula 10 wherein $R_1$ is a methyl group and $R_7$ is an acyl group is condensed with at least an equimolar amount of a silylated purine or pyrimidine base under catalysis, the protective groups present are cleaved off and the anomers of formula 16 formed are separated by crystallisation or chromatography using methods known per se.